# EUROPEAN PATENT SPECIFICATION

(11) **EP 4 243 710 B1**
(45) Date of publication and mention of the grant of the patent: **14.10.2026**
(21) Application number: 21892921.4
(22) Date of filing: 12.11.2021
(51) Int. Cl.: A61B 17/322, A61B 17/3205, A61B 17/34

(54) **SYSTEM FOR SECURING A NEEDLE OR GROUP OF NEEDLES WITHIN A SKIN GRAFTING SYSTEM**
SYSTEM ZUR BEFESTIGUNG EINER NADEL ODER EINER NADELGRUPPE IN EINEM HAUTTRANSPLANTATIONSSYSTEM
SYSTÈME DE FIXATION D'UNE AIGUILLE OU D'UN GROUPE D'AIGUILLES DANS UN SYSTÈME DE GREFFE DE PEAU

(30) Priority: 13.11.2020 US 202063113678 P
(43) Date of publication of application: 20.09.2023
(73) Proprietor: Medline Industries, LP, Springfield, IL 62703 (US)
(72) Inventor: ANDREW, Michael, A., Monona, WI 53716 (US); MEHNERT, John, Clayton, Madison, WI 53717 (US); FALENDYSZ, Gregory, Sun Prairie, WI 53590 (US); KERSTEN, Eric, J., Windsor, WI 53598 (US); GUILES, Marvin, A., Stow, MA 01775 (US)
(74) Representative: Maiwald GmbH
(86) International application number: PCT/US2021/059229
(87) International publication number: WO 2022/104130

(56) References cited:
- US-A1- 2015 258 319
- US-A1- 2015 290 444
- US-A1- 2017 281 878
- US-A1- 2019 223 908
- US-A9- 2007 293 884
- US-B2- 9 782 574

## Description

### CROSS-REFERENCE TO RELATED APPLICATIONS

The present application is based on, claims priority to, United States Provisional Patent Application No. 63/113,678, filed on November 13, 2020.

### BACKGROUND OF THE INVENTION

The subject matter disclosed herein generally relates to a needle-based skin grafting system and, more particularly, to systems and methods for managing and securing needles within a device for harvesting and scattering skin microcolumns.

An autograft can refer to tissue transplanted from one part of an individual's body (e.g., a "donor site") to another part (e.g., a "recipient site"). Autografts can be used, for example, to replace missing skin and other tissue and/or to accelerate healing resulting from trauma, wounds, burns, surgery and birth defects. Availability of tissue for autografting can be limited by characteristics of candidate donor sites, including a number and/or total area of tissue grafts, healing behavior of the donor site, similarity of the donor and recipient sites, aesthetic considerations, and the like.

Skin grafting can be performed surgically. For example, a conventional autograft procedure may include excision or surgical removal of burn injured tissue, choosing a donor site, which may be an area from which healthy skin is removed to be used as cover for the cleaned burned area, and harvesting, where the graft may be removed from the donor site (e.g., using an instrument similar to an electric shaver). Such instrument (e.g., a dermatome) can be structured to gently shave a thin piece of tissue (e.g., about 10/1000 of an inch (1 inch = 25,4 mm) thick for a split-thickness graft) from the skin at the undamaged donor site to use as a skin graft. The skin graft can then be placed over the cleaned wound to heal. Donor skin tissue can be removed to such a depth that the donor site can heal on its own, in a process similar to that of healing of a second degree burn.

Traditionally, sheet grafts and meshed grafts are the two types of autografts often used for a permanent wound coverage. A sheet graft can refer to a piece of skin tissue removed from an undamaged donor site of the body, in a process that may be referred to as harvesting. The size of the donor skin piece that is used may be about the same size as the damaged area. The sheet graft can be applied over the excised wound, and stapled or otherwise fastened in place. The donor skin tissue used in sheet grafts may not stretch significantly, and a sheet graft can be obtained that is slightly larger than the damaged area to be covered because there may often be a slight shrinkage of the graft tissue after harvesting.

Sheet grafts can provide an improved appearance of the repaired tissue site. For example, sheet grafts may be used on large areas of the face, neck and hands if they are damaged, so that these more visible parts of the body can appear less scarred after healing. A sheet graft may be used to cover an entire burned or damaged region of skin. Small areas of a sheet graft can be lost after placement because a buildup of fluid (e.g., a hematoma) can occur under the sheet graft following placement of the sheet graft.

A meshed skin graft can be used to cover larger areas of open wounds that may be difficult to cover using sheet grafts. Meshing of a skin graft can facilitate skin tissue from a donor site to be expanded to cover a larger area. It also can facilitate draining of blood and body fluids from under the skin grafts when they are placed on a wound, which may help prevent graft loss. The expansion ratio (e.g., a ratio of the unstretched graft area to the stretched graft area) of a meshed graft may typically be between about 1:1 to 1:4. For example, donor skin can be meshed at a ratio of about 1:1 or 1:2 ratio, whereas larger expansion ratios may lead to a more fragile graft, scarring of the meshed graft as it heals, and/or extended healing times.

A conventional graft meshing procedure can include running the donor skin tissue through a machine that cuts slits through the tissue, which can facilitate the expansion in a pattern similar to that of fish netting or a chain-link fence. Healing can occur as the spaces between the mesh of the stretched graft, which may be referred to as gaps or interstices, fill in with new epithelial skin growth. However, meshed grafts may be less durable graft than sheet grafts, and a large mesh can lead to permanent scarring after the graft heals.

As an alternative to autografting, skin tissue obtained from recently-deceased people (which may be referred to, e.g. as a homograft, an allograft, or cadaver skin) can be used as a temporary cover for a wound area that has been cleaned. Unmeshed cadaver skin can be put over the excised wound and stapled in place. Post-operatively, the cadaver skin may be covered with a dressing. Wound coverage using cadaveric allograft can then be removed prior to permanent autografting.

A xenograft or heterograft can refer to skin taken from one of a variety of animals, for example, a pig. Heterograft skin tissue can also be used for temporary coverage of an excised wound prior to placement of a more permanent autograft, and may be used because of a limited availability and/or high expense of human skin tissue. In some cases religious, financial, or cultural objections to the use of human cadaver skin may also be factors leading to use of a heterograft. Wound coverage using a xenograft or an allograft is generally a temporary procedure which may be used until harvesting and placement of an autograft is feasible.

Recently, needle-based tissue harvesting has been shown to be an advantageous alternative to sheet or blade-based procedures. Needle-based harvesting presents extensive advantages over sheet or blade-based procedures, such as reduction in the complexity and complications associated with harvesting and deployment of tissue, reduced tissue required from donor sites, reduced or scarring at the donor site, and many others. However, to realize these advantages, the harvesting needles must be carefully controlled. For example, when harvesting tissue via needles, the number of needles used and/or the depth to which the needles can be pushed into the skin is correlated to resulting impact on the donor site and, thereby, the time for healing at the donor site. Accordingly, even small improvements in systems and methods for managing, securing, and/or deploying, needles can yield appreciable benefits.

Document US 2015/290444 A1 relates to an applicator for applying a micro needle.

Document US 2015/258319 A1 relates to a force-controlled applicator for applying a micro needle device to skin.

### BRIEF DESCRIPTION OF THE DISCLOSURE

The present invention discloses a skin grafting system as defined by independent claim 1, with further embodiments defined by the dependent claims.

The present disclosure provides systems and methods for managing and securing the position of needles with respect to needle-based tissue harvesting.

The present invention provides a skin grafting system that includes a plurality of hollow microneedles actuatable between a retracted position and an extended position. The system further includes a rigid member coupled to a plurality of hollow microneedles, and a latch assembly having at least one latch coupled to the latch assembly. The at least one latch is configured to inhibit movement of the rigid member when the plurality of hollow microneedles are in the extended position.

In another aspect, the present disclosure provides a skin grafting system including a carrier actuatable between a retracted position and an extended position. The system further includes a plurality of hollow microneedles coupled to the carrier and configured to extract tissue cores from a donor site as the carrier moves from a retracted position to an extended position and back to a retracted position. Additionally, the system includes a latch configured to move between a plurality of positions, including a latched position restricting movement of the carrier from the extended position to the retracted positon to thereby lock the plurality of hollow microneedles in a position configured to engage the donor site.

In another aspect, the present disclosure provides a system for securing a plurality of microneedles during a skin grafting process. The system includes a rigid member coupled to a proximal end of the plurality of microneedles, and a latch assembly. The latch assembly includes at least one pair of latches, each latch moveably coupled to the latch assembly and configured to engage the rigid member. The latch assembly further includes a spring disposed between the at least one pair of latches, and configured to bias the at least one pair of latches to a latched position. The at least one pair of latches are configured to inhibit movement of the rigid member when in the latched position.

The following description and the accompanying drawings set forth in detail certain illustrative embodiments of the present disclosure. However, these embodiments are indicative of but a few of the various ways in which the principles of the disclosure can be employed. Other embodiments and features will become apparent from the following detailed description of the present disclosure when considered in conjunction with the drawings.

### BRIEF DESCRIPTION OF THE DRAWINGS

The descriptions hereafter are provided with reference to the accompanying drawings, wherein like reference numerals denote like elements.
FIG. 1 is a top perspective view of a skin grafting system, including a cartridge, in accordance with some implementations of the present disclosure.
FIG. 2A is a front perspective view of the system of FIG. 1.
FIG. 2B is a top view of a user interface that may be included in the system of FIG. 2A, in accordance with some implementations of the present disclosure.
FIG. 3A is a cutaway view of the handheld device of FIG. 2A, in accordance with some implementations of the present disclosure.
FIG. 3B is a cutaway view of a housing corresponding to the handheld device of FIG. 2A, in accordance with some implementations of the present disclosure.
FIG. 4A is a rear perspective view of an internal drive assembly and related elements corresponding to the handheld device of FIG. 2A, in accordance with some implementations of the present disclosure.
FIG. 4B is a right perspective view of a left frame assembly corresponding to the internal assembly of FIG. 4A, in accordance with some implementations of the present disclosure.
FIG. 4C is a right perspective view of a right frame assembly corresponding to the internal assembly of FIG. 4A, in accordance with some implementations of the present disclosure.
FIG. 4D is a rear perspective view of a horizontal component assembly corresponding to the internal assembly of FIG. 4A, in accordance with some implementations of the present disclosure.
FIG. 4E is a rear perspective view of a vertical component assembly corresponding to the internal assembly of FIG. 4A, in accordance with some implementations of the present disclosure.
FIG. 4F is a block diagram of a lockdown latch assembly corresponding to the internal assembly of FIG. 4A, in accordance with some implementations of the present disclosure.
FIG. 5A is a perspective view of a cartridge assembly including a removable cover, in accordance with some implementations of the present disclosure.
FIG. 5B is a perspective view of a cartridge loaded into a reusable handheld device, corresponding to the cartridge of FIG. 5A, in accordance with some implementations of the present disclosure.
FIG. 5C is an image of the cartridge of FIG. 5A, in accordance with some implementations of the present disclosure.
FIG. 6A is an example of a microneedle and pin assembly that can harvest tissue, in accordance with some implementations of the present disclosure.
FIG. 6B is a perspective view of a microneedle and pin assembly that can harvest tissue, in accordance with some implementations of the present disclosure.
FIG. 6C is a plan view of a microneedle array, in accordance with some implementations of the present disclosure.
FIG. 7A is an exploded view of a lockdown latch assembly, in accordance with some implementations of the present disclosure.
FIG. 7B is a cross-sectional view of the lockdown latch assembly of FIG. 7A in a latched position, in accordance with some implementations of the present disclosure.
FIG. 7C is a cross-sectional view of the lockdown latch assembly of FIG. 7A in an unlatched position, in accordance with some implementations of the present disclosure.
FIG. 8A is an exploded view of another lockdown latch assembly, in accordance with some implementations of the present disclosure.
FIG. 8B is a cross-sectional view of the lockdown latch assembly of FIG. 8A in a latched position, in accordance with some implementations of the present disclosure.
FIG. 8C is a cross-sectional view of the lockdown latch assembly of FIG. 8A in an unlatched position, in accordance with some implementations of the present disclosure.
FIG. 9A is an exploded view of another lockdown latch assembly, in accordance with some implementations of the present disclosure.
FIG. 9B is a perspective view of a latch assembly of the lockdown latch assembly of FIG. 9A, in accordance with some implementations of the present disclosure.
FIG. 9C is a cross-sectional view of the lockdown latch assembly of FIG. 9A in a latched position, in accordance with some implementations of the present disclosure.
FIG. 9D is a cross-sectional view of the lockdown latch assembly of FIG. 9A in an unlatched position, in accordance with some implementations of the present disclosure.
FIG. 10A is an exploded view of another lockdown latch assembly, in accordance with some implementations of the present disclosure.
FIG. 10B is a bottom perspective view of the lockdown latch assembly of FIG. 10A, in accordance with some implementations of the present disclosure.
FIG. 10C is a cross-sectional view of the lockdown latch assembly of FIG. 10A in a latched position, in accordance with some implementations of the present disclosure.
FIG. 10D is a cross-sectional view of the lockdown latch assembly of FIG. 10A in an unlatched position, in accordance with some implementations of the present disclosure.
FIG. 11 is a procedural flowchart illustrating a method of harvesting and scattering tissue, in accordance with some implementations of the present disclosure.

### DETAILED DESCRIPTION OF THE DISCLOSURE

The following discussion is presented to enable a person skilled in the art to make and use the systems and methods of the present disclosure. Various modifications to the illustrated embodiments will be readily apparent to those skilled in the art, and the high-level principles herein can be applied to other embodiments and applications without departing from embodiments of the present disclosure. Thus, embodiments of the present disclosure are not intended to be limited to embodiments shown, but are to be accorded the widest scope consistent with the principles and features disclosed herein.

The detailed description is to be read with reference to the figures. The figures depict selected embodiments and are not intended to limit the scope of embodiments of the present disclosure. Skilled artisans will recognize the examples provided herein have many useful alternatives and fall within the scope of embodiments of the present disclosure. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings. As used herein, unless expressly stated otherwise, "connected" means that one element/feature is directly or indirectly connected to another element/feature, and not necessarily electrically or mechanically. Likewise, unless expressly stated otherwise, "coupled" means that one element/feature is directly or indirectly coupled to another element/feature, and not necessarily electrically or mechanically.

Embodiments of the present disclosure may be described herein in terms of functional and/or logical block components and various processing steps. It should be appreciated that such block components may be realized by any number of hardware, software, and/or firmware components configured to perform the specified functions. For example, an embodiment may employ various integrated circuit components, e.g., digital signal processing elements, logic elements, diodes, etc., which may carry out a variety of functions under the control of one or more processors or other control devices. Other embodiments may employ program code, or code in combination with other circuit components.

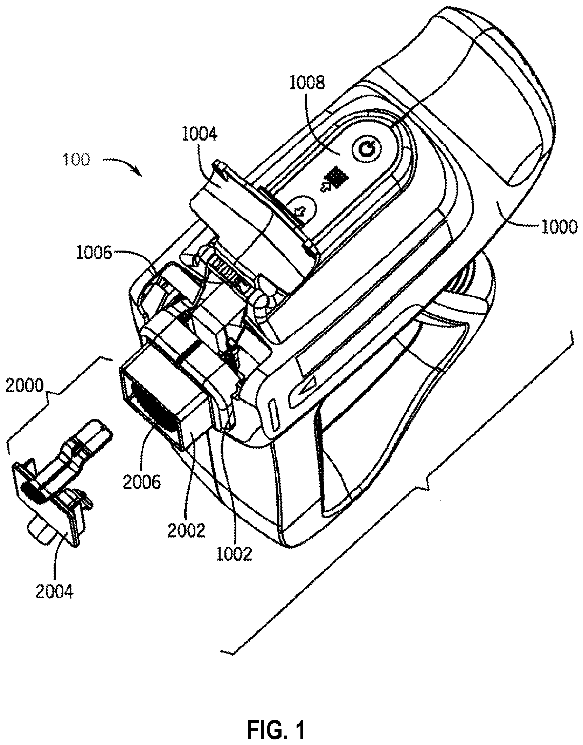

Referring now to FIG. 1, a skin grafting system 100 is shown, in accordance with some implementations of the present disclosure. In some configurations, the skin grafting system 100 can be configured to harvest and scatter donor tissue. As shown, the skin grafting system 100 can include a handheld device 1000 (which can be reusable) and a cartridge assembly 2000. As will be described in greater detail below, the cartridge assembly 2000 can include a cartridge housing 2002 and a cartridge cover 2004. The cartridge assembly 2000 can include a needle and pin array 2006, according to some configurations. In the illustrated configuration, the needles can be configured as microneedles. Notably, the cartridge assembly 2000 can include a simplified microneedle array 2006 (i.e., without pins).

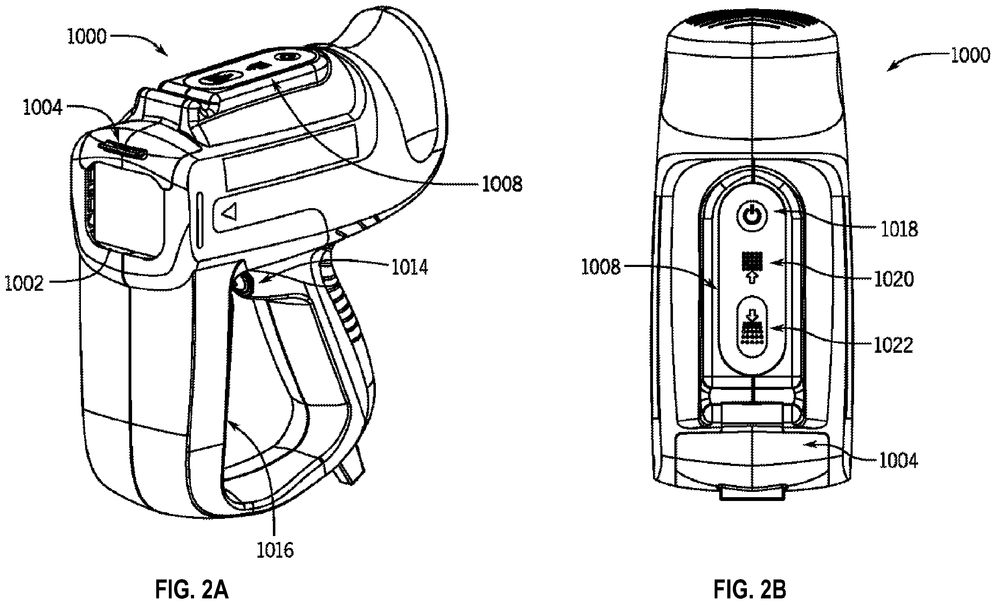

As shown by FIGS. 1-2B, the handheld device 1000 can include an engagement slot 1002 configured to receive the cartridge assembly 2000. A loading door 1004 can move between an "open" position (see, e.g., FIG. 1) and a "closed" position (see, e.g., FIGS. 2A-2B). In some configurations, the loading door 1004 can be hinged and further configured to open and close over a loading aperture 1006. The handheld device 1000 can include a door sensor, which can determine the position of the loading door 1004. The loading aperture 1006 can be sized such that the cartridge assembly 2000 can slide in and out of the engagement slot 1002, as desired by the user. Advantageously, the cartridge assembly 2000 can be single-use and/or disposable (including, for example, multiple uses for a single patient), while the handheld device 1000 can be designed to be multi-use. As shown by FIG. 2A, the handheld device 1000 can further include a trigger 1014. The trigger 1014 can be configured to activate a harvesting process and/or a scattering process in response to selection via a user interface 1008 and/or trigger inputs by a user. In some configurations, the handheld device 1000 can include an indicator light 1016. The indicator light 1016 can be positioned such that a user can readily view the indicator light 1016 during harvesting and/or scattering.

In some configurations, the handheld device 1000 can include a user interface 1008. As shown, the user interface 1008 can include a stand-by input 1018, an indicator light 1020, and/or a scatter input 1022. In some configurations, the indicator light 1020 can operate the same as, or similar to, the indicator light 1016 (as described above). The stand-by input 1018, the indicator lights 1016,1020, and the scatter input 1022 can provide visual feedback to a user that correspond to current operation of the skin grafting system 100 as the skin grafting system 100 is utilized according to a skin grafting process, such as will be described.

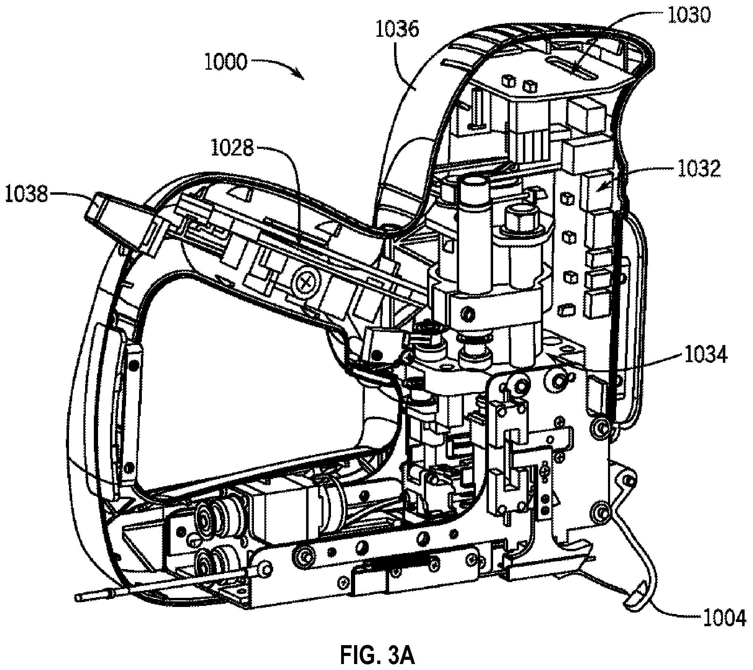

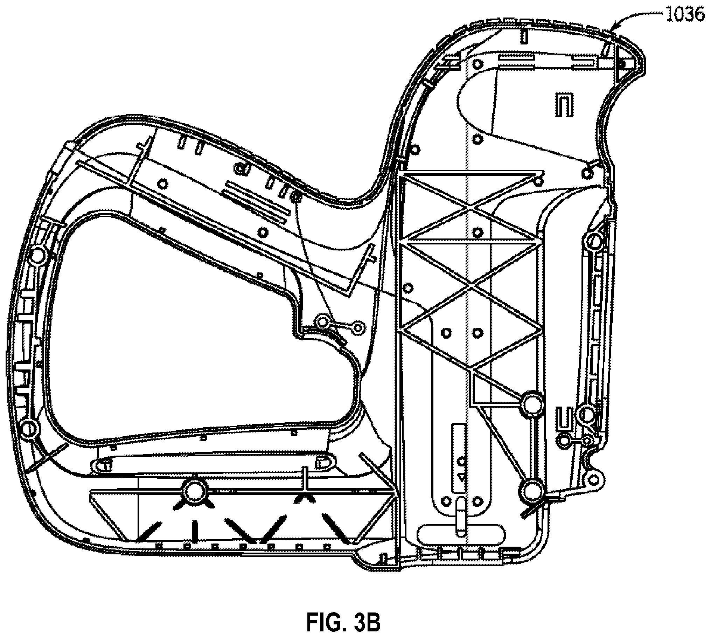

Referring now to FIGS. 3A-3B, cutaway views of the handheld device 1000 are shown, according to configurations of the present disclosure. The handheld device 1000 is shown to include various internal controllers. In some configurations, the handheld device 1000 can include a power module 1028, an actuator controller 1030, and/or a main controller 1032. The power module 1028 can be in electrical communication with a power input 1038. In some configurations, a drive system can include an actuator in communication with the actuator controller 1030.

Still referring to FIGS. 3A-3B, in some configurations, the handheld device 1000 can include a housing 1036. The housing 1036 can include a left enclosure half and a right enclosure half. In some configurations, each of the left enclosure half, the right enclosure half, the loading door 1004 and the enclosure mount cover can be individually injection molded. The left and right enclosure halves can be made up of a hard plastic substrate, and in some configurations, a softer elastomeric over-molded section. Similarly, the loading door 1004 and the enclosure mount cover can be made up of hard plastic substrate. In some configurations, the interior of the housing 1036 can interface with internal subassemblies. As an example, ribs can be affixed to the interior of the housing 1036, and can be configured to support various printed circuit boards (PCBs). The ribs can separate the PCBs (e.g., power module 1028, actuator controller 1030, and main controller 1032) from internal moving components. Additionally, in some configurations, the housing 1036 can support the internal subassembly 1034 via pins and vibration damping boots. This can dampen the operational impacts of the internal subassembly 1034 (e.g., from a user, from internal moving components), as well as protect the internal subassembly 1034 from damage due to external impacts (e.g., from dropping the handheld device 1000).

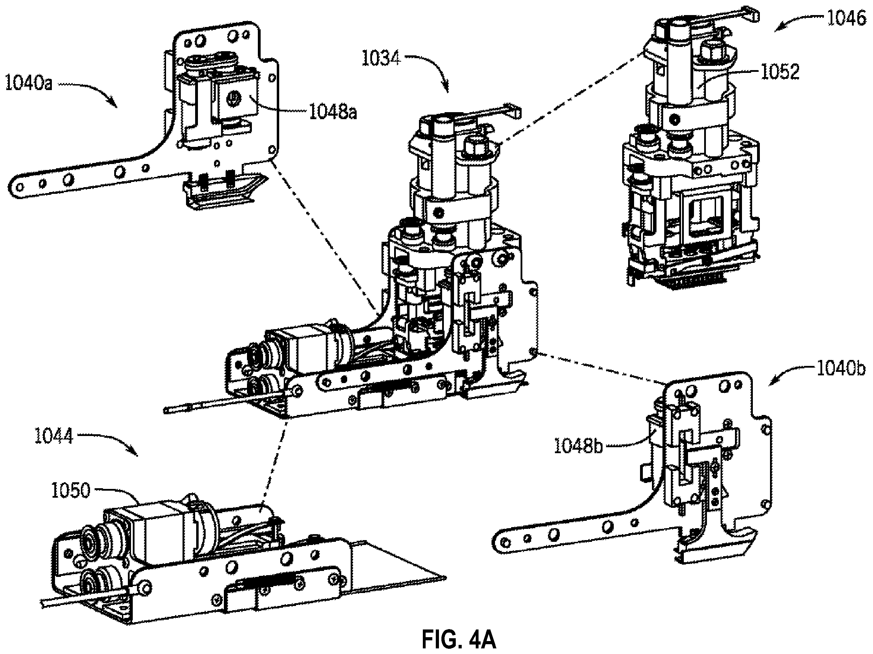

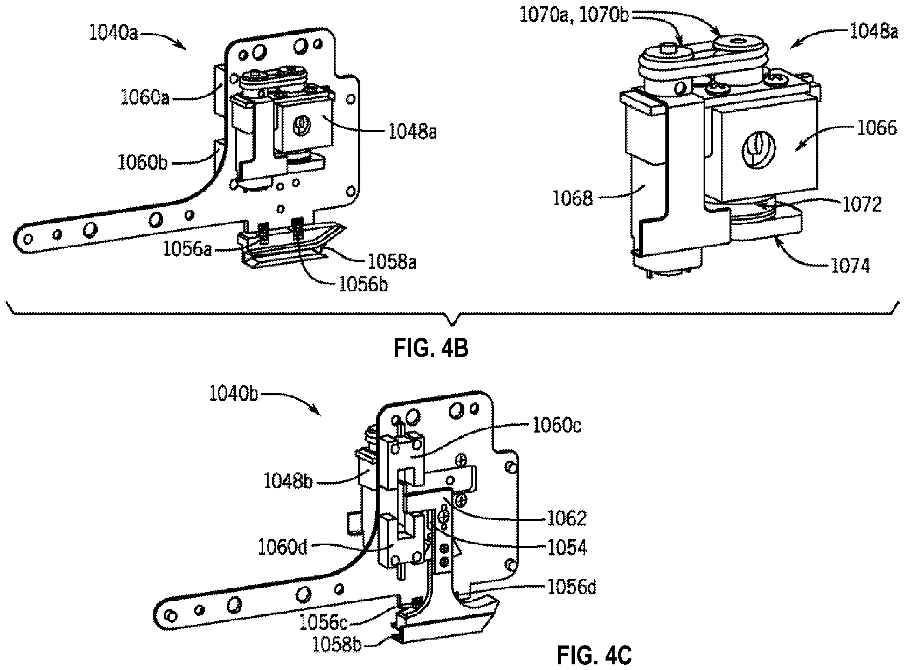

Referring now to FIGS. 4A-4E, various internal assemblies corresponding to handheld device 1000 are shown, according to some configurations. FIG. 4A shows the internal subassembly 1034 that can include a left frame assembly 1040a, a right frame assembly 1040b, a horizontal component assembly 1044, and/or a vertical component assembly 1046. Each of the left and right frame assemblies 1040a, 1040b can include a corresponding flipper assembly (e.g., left flipper assembly 1048a, right flipper assembly 1048b). In some configurations, the horizontal component assembly 1044 can include a horizontal motor 1050. Further, the vertical component assembly 1046 can include an actuator 1052. In some configurations, the actuator 1052 can be an electromagnetic actuator (e.g., a solenoid). In other configurations, the actuator 1052 can be a linear actuator. In yet further configurations, the actuator 1052 can be any one of a mechanical, hydraulic, pneumatic, or electrical actuator. Those skilled in the art will readily recognize other forms of actuators that could be utilized in the vertical component assembly to apply or transfer an actuation force to another component therein.

Still referring to FIGS. 4A-4E, and in particular FIGS. 4B-4C, further exemplary details of the left and right frame assemblies 1040a, 1040b are shown, according to some configurations. In some configurations, the left frame assembly 1040a and the right frame assembly 1040b can be the same or substantially similar (e.g., symmetrical). As shown, the left frame assembly 1040a can include a left flipper assembly 1048a affixed to a first side of a left frame. Additionally, the left frame assembly 1040a can include flag sensors 1060a, 1060b, affixed to a second side of the left frame. The flag sensors 1060a, 1060b can communicate with a position sensing linear slide 1054, and a position sensing flag 1062. In some configurations, the left frame assembly 1040a can include position sensing springs 1056a, 1056b, which can contact a tissue interface 1058a. The tissue interface 1058a can be positioned on a third side of the left frame. In some configurations, the left frame assembly 1040a can attach to a portion of the vertical component assembly 1046 via screws and alignment pins, or other attachment systems.

In some configurations, the right frame assembly 1040b can include flag sensors 1060c, 1060d, affixed to a first side of a right frame. The flag sensors 1060c, 1060d can communicate with a position sensing linear slide 1054, and a position sensing flag 1062. Additionally, as shown, the right frame assembly 1040b can include a right flipper assembly 1048b affixed to a second side of the right frame. In some configurations, the right frame assembly 1040b can include position sensing springs 1056c, 1056d, which can contact a tissue interface 1058b. The tissue interface 1058b can be positioned on a third side of the right frame. In some configurations, the right frame assembly 1040b can attach to a portion of the vertical component assembly 1046 via screws and alignment pins.

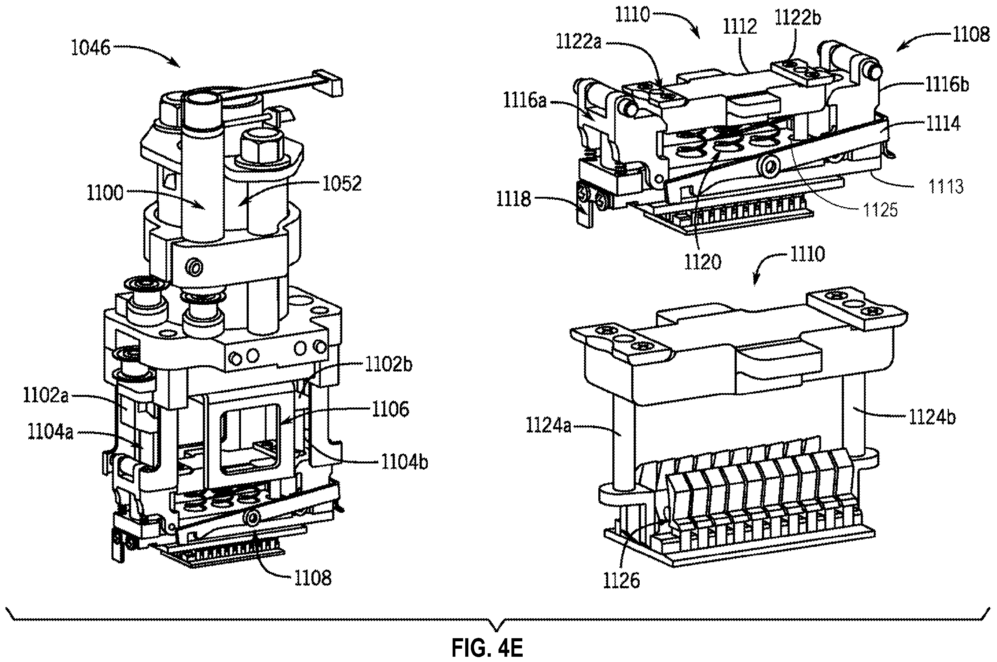

The flipper assemblies 1048a, 1048b can include a flipper mounting block 1066, and a flipper motor 1068. In some configurations, the flipper mounting block 1066 can be constructed from a dielectric material. The flipper motor 1068 can be connected to (and control) flipper driver pulleys 1070a, 1070b. A bearing (e.g., a thrust bearing) 1072 can support an axial load exerted by the needle top plate (e.g., needle top plate 1112 as described below) on a flipper 1074. The flipper 1074 can rotate in accordance with motor actuation, and the flipper driver pulleys 1070a, 1070b can prevent any downward movement of the flipper 1074 during operation of the handheld device 1000. In some configurations, the flipper 1074 can include two connected components, such as two brass components that are brazed together. In some configurations, the flipper 1074 can include two components formed from stainless steel and coupled together with one or more fasteners. The primary function of the flipper 1074 can be to hold a needle top plate 1112 of Fig. 4E in place when loading needle retract springs. The flipper 1074 can then move out of the way of the needle top plate 1112 during the remainder of normal operation. In some configurations, the flipper mounting block 1066 can act as a guide for actuator plunger bar 1106 of Fig. 4E (e.g., to keep proper alignment).

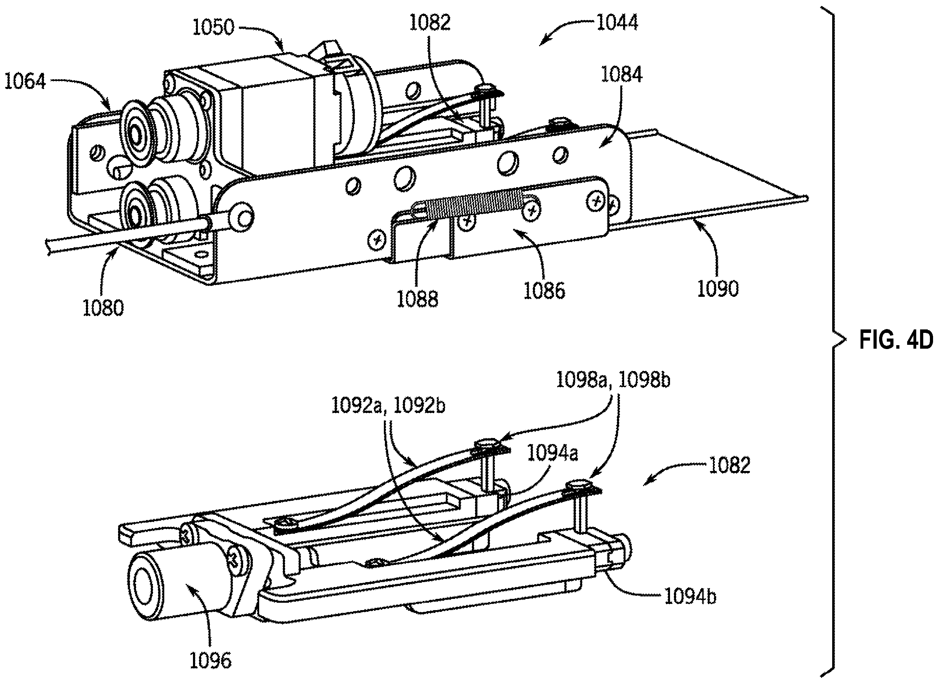

Still referring to FIGS. 4A-4E, and in particular FIG. 4D, further exemplary details of the horizontal component assembly 1044 are shown, according to some configurations. The horizontal component assembly can include sensors, actuators, and/or guides for positioning a horizontal carriage assembly 1082 and, thereby, the hammers 1098a, 1098b used to drive microneedles into the tissue (as will be described below). In some configurations, a horizontal flag sensor 1064 can be used to position the horizontal component assembly 1082. As shown, the horizontal component assembly 1044 can include the horizontal carriage assembly 1082 that can be configured to mount the horizontal motor 1050. In some configurations, a horizontal chassis 1084 can support the horizontal carriage assembly 1082. Additionally, the right frame assembly 1040b and the left frame assembly 1040a can be affixed to opposing sides of the horizontal chassis 1084, for example, using rivets. An earth-ground connection 1080 can be attached to the horizontal chassis 1084, according to some configurations.

In some configurations, the horizontal component assembly 1044 can further include a retractable slide door 1090. The slide door 1090 can extend across the loading aperture 1006 when the cartridge assembly 2000 has not been inserted into the engagement slot 1002. Accordingly, a user can be prevented from placing anything into the handheld device 1000 during the absence of the cartridge assembly 2000. The sliding door 1090 can be secured to the horizontal chassis 1084 via a sliding door mount 1086, which can be affixed to the horizontal chassis 1084. Additionally, a sliding door spring 1088 can be secured to the sliding door mount 1086, and biased such that the slide door 1090 remains in a "closed" position (i.e., extended across the loading aperture 1006) when a cartridge is not loaded.

As shown, the horizontal carriage assembly 1082 can include hammers 1098a, 1098b, corresponding hammer return springs 1092a, 1092b, and corresponding hammer guides 1094a, 1094b, according to some configurations. Generally, the horizontal carriage assembly 1082 can be configured to position and guide the hammers 1098a, 1098b to drive the microneedles into the tissue. In some configurations, the hammer guides 1094a, 1094b can be made of bronze, which can help to maintain bearing surfaces throughout many harvesting and scattering cycles. Additionally, in some configurations, the hammers 1098a, 1098b can be hardened 17-4 stainless steel, which can provide superior wear characteristics while maintaining anti-corrosion properties. Alternatively, the hammers 1098a, 1098b can be a different bearing material. The horizontal carriage assembly 1082 can further include a horizontal leadscrew drive nut 1096. Additionally, the horizontal leadscrew assembly 1096 can be a Teflon-coated lead screw, and an acetal drive nut designed to reduce friction. Alternatively, the horizontal leadscrew assembly 1096 can include other material types. The horizontal leadscrew assembly 1096 can provide a pitch adequate for positional resolution and linear force. The horizontal carriage assembly 1082 can additionally use motor stalling to sense whether or not a cartridge is loaded, or if there is a handheld device jam.

Still referring to FIGS. 4A-4E, and in particular FIG. 4E, further exemplary details of the vertical component assembly 1046 are shown, according to some configurations. As shown, the vertical component assembly 1046 can include the actuator 1052 and corresponding actuator plunger bar 1106. Additionally, the vertical component assembly 1046 can include a vertical motor 1100, and associated unlock cams 1102a, 1102b and vertical leadscrews 1104a, 1104b. In some configurations, the vertical position of the vertical carriage subassembly 1108 can be controlled by traveling up and down on the vertical leadscrews 1104a, 1104b (e.g., using the vertical motor 1100). As will be described, vertical positioning can move each of the microneedles corresponding to the cartridge assembly 2000. In general, the vertical component assembly 1046 can be configured to interface with and manipulate the cartridge assembly 2000 and its associated components during harvesting and/or scattering of tissue. In some configurations, the vertical motor 1100 can be sized to fit within the vertical component assembly 1046 while still providing the torque and speeds necessary for manipulating the microneedle positions.

In some configurations, the actuator 1052 can deliver an operating force to the hammers 1098a, 1098b during harvesting. In configurations where the actuator 1052 is in the form of a solenoid, the actuator 1052 can be activated by a half wave of AC current, as one non-limiting example. The force delivered by the actuator 1052 can increase sharply, towards the end of its stroke. In some configurations, the mass of the actuator plunger bar 1106 and the actuator plunger can be selected based on the energy needed to drive the microneedles into the tissue. In some configurations, a stop (e.g., a brass stop) can be integrated into the actuator 1052, which can enable extension control of the actuator plunger bar 1106 and absorption of remaining kinetic energy at the end of the stroke.

In some configurations, the vertical component assembly 1046 can include a vertical carriage assembly 1108. As shown, the vertical carriage assembly 1108 can include a needle retract slide 1110 with a top plate 1112. In some configurations, opposite ends of the vertical carriage assembly 1108 can include needle retract slide-latches 1116a, 1116b with corresponding latch plates 1122a, 1122b. The latch plates 1122a, 1122b can define a maximum or locked position of the needle retract slide 1110. Additionally, needle retract springs 1120 can be integrated into the vertical carriage assembly 1108, such that efficient retraction of the microneedles can be achieved over the pins. The needle retract springs 1120 can be arranged between the top plate 1112 and a vertical carriage body 1113. The needle retract slide-latches 1116a, 1116b can be used to lock down the needle retract slide 1110 in preparation for harvesting. The vertical carriage assembly 1108 can also move both the needles and pins (e.g., pins within the microneedles) at the same time.

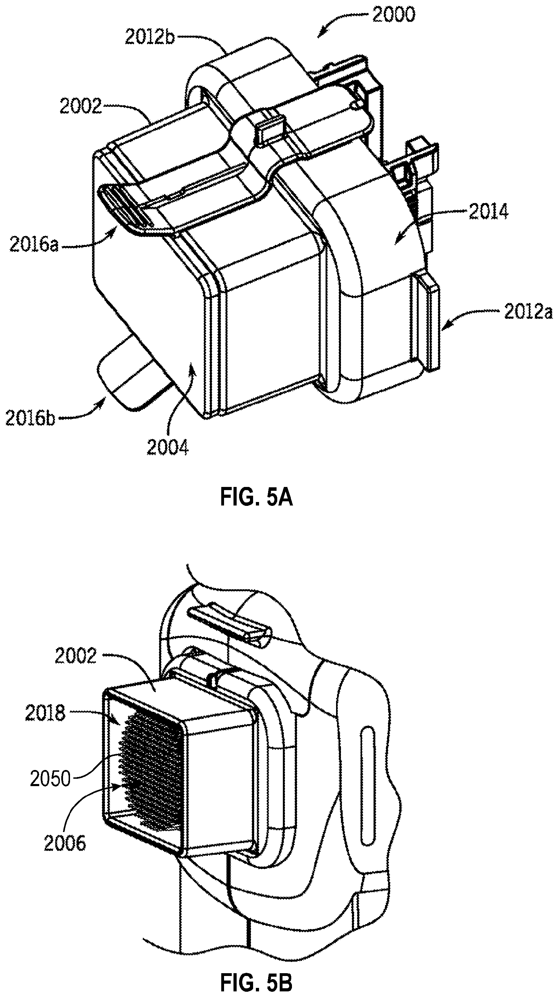

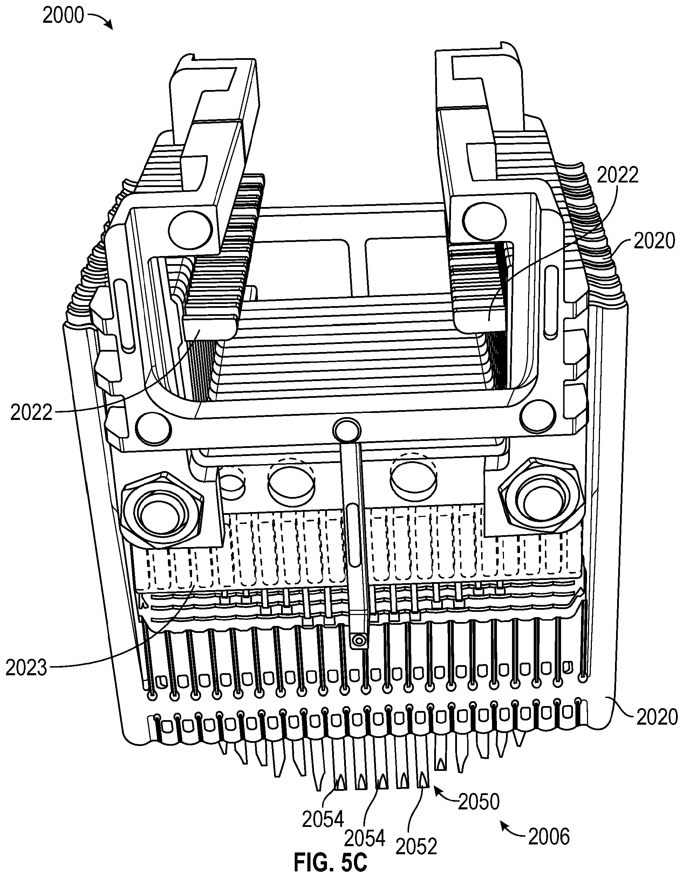

In some configurations, the vertical carriage assembly 1108 can include a cartridge latch 1114, which can be configured to secure the cartridge assembly 2000 upon insertion into the loading aperture 1006. Additionally, a vertical flag 1118 can be affixed to the exterior of the vertical carriage assembly 1108, according to some configurations, or integrally formed into the vertical carriage body 1113. As shown, the needle retract slide 1110 can further include guideposts 1124a, 1124b, which can be configured to guide the needle retract slide 1110 during vertical movement. As will be described herein, the needle retract slide 1110 can include a lockdown latch assembly 1126, which can be in contact with the guideposts 1124a, 1124b, and configured to engage and disengage the microneedles during operation of the handheld device 1000. The needle retract slide 1110 can be a spring loaded subassembly that serves at least two purposes. First, the slide 1110 can lock needle plates 2020 (see, e.g., FIG. 5C) down (after being driven into the tissue). Second, the slide 1110 can retract the needles. In some configurations, the needle retract slide 1110 is only capable of retracting the needles, and cannot move the microneedles forward. Additionally, in some configurations, the lockdown latch assembly 1126 may be only functional after the skin grafting system 100 has gone through initialization. Further detail regarding the operation of the skin grafting system 100 is provided below.

Referring now to FIGS. 5A-5C, the cartridge assembly 2000 and cartridge housing 2002 are shown, according to some configurations. As shown, the cartridge assembly 2000 can include the cartridge housing 2002, and a cartridge cover 2004 that can be removably affixed to a microneedle chamber 2018. The microneedle chamber 2018 can enclose the array of microneedles 2006 including a plurality of microneedles. In some configurations, the microneedles can be arranged as an array within the microneedle chamber 2018. As shown by FIG. 5A, the combination of the cartridge cover 2004 and the microneedle chamber 2018 can form an enclosure for the array of microneedles 2006. The cartridge cover 2004 can include release levers 2016a, 2016b, which can be simultaneously depressed by a user to remove the cartridge cover 2004 from being engaged with the cartridge housing 2002.

In some configurations, the cartridge assembly 2000 can include a tissue stabilizer 2014, which forms a peripheral housing and can be configured to stabilize tissue during harvesting. That is, the tissue stabilizer 2014 forms a peripheral housing that is wider than the microneedle chamber 2018, allowing for a greater distribution of force during use of the skin grafting system 100 on tissue. According to the illustrated configuration, the tissue stabilizer extends away from the cartridge housing 2002. As shown, the tissue stabilizer 2014 can further include loading tabs 2012a, 2012b that extend outwardly. In some configurations, the loading tabs 2012a, 2012b can slide into contact with the engagement slot 1002 during loading of the cartridge assembly 2000 into the loading aperture 1006.

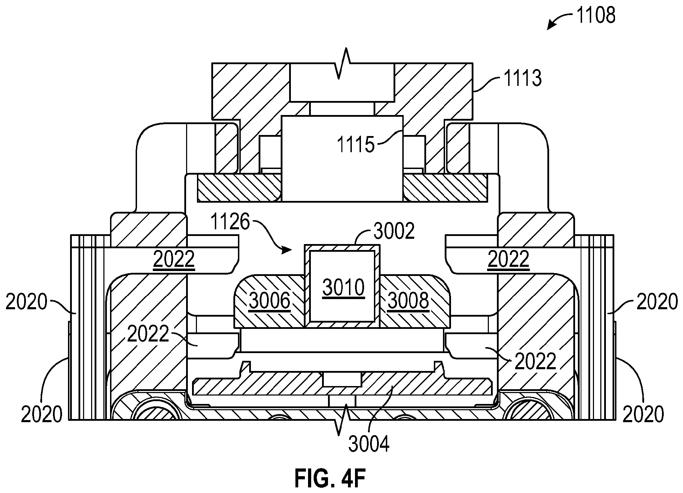

With reference to FIG. 5C, the cartridge assembly 2000 is shown without the tissue stabilizer 2014. In some configurations, the cartridge assembly 2000 can include one or more needle carriers. In the illustrated configuration, the needle carriers are configured as needle plates (e.g., needle segments) 2020 that are slidably within the cartridge assembly 2000 and moveable between an extended position (not shown) and a retracted position (as shown in FIG. 5C). As shown, one or more of the plurality of microneedles 2050 can be coupled to each of the needle plates 2020, thereby forming a row of microneedles on each needle plate 2020. In some configurations, the needle plates 2020 can also include a rigid member. In the illustrated configuration, the rigid member can be in the form of a pair of arms 2022 extending horizontally inward from opposing lateral sides of the needle plate 2020, although other configurations are also envisioned. For example, the rigid members of the needle plates can be arms that extend horizontally outwards from opposing lateral sides of the needle plate. In some configurations, the rigid members may be non-horizontal arms, where the arms angle upwards or downwards. In other configurations, the rigid members may not be arms, and may instead be another mechanical feature or structure (e.g., hooks, loops, eyelets, plates, protrusions, etc.). As will be described herein, the arms 2022 can be configured to engage with the lockdown latch assembly 1126 (FIG. 4F) to lock the needle plates 2020, and thus the microneedles 2050, in the extended position. The locking of the needle plates 2020 can prevent the microneedles 2050 from retracting during a harvest process.

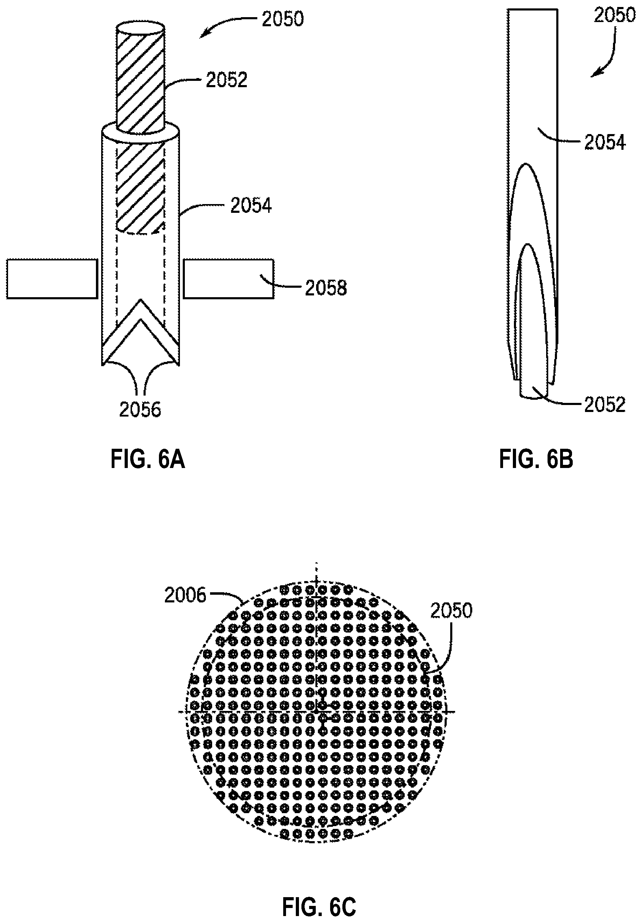

Referring now to FIGS. 6A-6C, a microneedle 2050 and a microneedle array 2006 are shown, according to configurations of the present disclosure. The microneedle 2050 can facilitate harvesting of tissue cores from a donor site. In some configurations, the microneedle 2050 can be configured as a hollow microneedle and can include a hollow tube 2054 that can include a plurality of points 2056 at the distal end thereof. In some non-limiting examples, needle systems such as described in US Patent Nos. 9,060,803; 9,827,006; 9,895,162; and US Patent Application Publication Nos. 2015/0216545; 2016/0015416; 2018/0036029; 2018/0140316 and/or combinations or components thereof may be used.

In some configurations of the present disclosure, the hollow tube 2054 can be provided with two points 2056, and the points 2056 can be sufficiently angled for penetrating and cutting the biological tissue cores to remove small micrografts in the form of a tissue column therefrom. Such a hollow tube 2054 can be provided with two points 2056, and a "narrow heel" portion positioned between the two points 2056. According to some configurations, the narrow heel portion can be sharpened, such that a cutting edge corresponding to the hollow tube 2054 is created.

In some configurations, the hollow tube 2054 can be slidably attached to a substrate 2058, such that the hollow tube 2054 can pass through a hole provided in the substrate 2058, as shown in FIG. 6A. The position of the hollow tube 2054 relative to the substrate 2058 can be controlled by translating the hollow tube 2054 relative to the substrate 2058, e.g., substantially along the longitudinal axis of the hollow tube 2054. In this manner, the distance that the distal end of the hollow tube 2054 protrudes past the lower surface of the substrate 2058 can be controllably varied.

Referring now to FIGs. 5C-6C, the cartridge assembly 2000 can further include a pin 2052 provided in the central lumen or opening of the hollow tube 2054 of each of the plurality of microneedles 2050. The cartridge assembly 2000 can also include one or more pin carriers. In the illustrated configuration, the pin carriers are configured as pin plates 2023 that coupled to the frame of the cartridge assembly 2000 such that the pins 2052 are fixed and retained by the pin plate 2023, so that the hollow tubes 2054 can move independently from the pins 2052 (see, e.g., FIG. 5C). As shown, one or more of a plurality of pins 2052 can be coupled to each of the pin plates 2023, thereby forming a row of pins on each pin plate 2023. According to the illustrated configuration, each pin 2052 corresponds to a hollow tube 2054, such that that each microneedle 2050 in the needle array 2006 includes a corresponding pin 2052.

The diameter of the pin 2052 can be substantially the same as the inner diameter of the hollow tube 2054 or slightly smaller, such that the hollow tube 2054 can be translated along an axis corresponding to pin 2052 while the pin 2052 fills or occludes most or all of the inner lumen of the hollow tube 2054. The pin 2052 can be formed of a low-friction material, or coated with a low-friction material such as, e.g., Teflon^{®} or the like, to facilitate motion of the hollow tube 2054 with respect to the pin 2052 and/or inhibit accumulation or sticking of biological material to the pin 2052. According to some configurations, the pins can be formed from 17-7 stainless steel and the needles can be formed from 303 stainless steel. The distal end of the pin 2052 can be substantially flat to facilitate displacement of a tissue core within the hollow tube 2054, when the hollow tube 2054 is translated relative to the pin 2052.

The hollow tube 2054 can be translated relative to the pin 2052, e.g., substantially along the longitudinal axis of the hollow tube 2054. In this manner, the position of the distal end of the hollow tube 2054 relative to that of the distal end of the pin 2052 can be controllably varied. For example, the location of the distal ends of both the hollow tube 2054 and the pin 2052 relative to that of the lower surface of the substrate 2058 can be controllably and independently selected and varied.

FIG. 6B shows one configuration of the present disclosure, in which the pin 2052 can be positioned relative to the hollow tube 2054 such that their distal ends are substantially aligned. In another configuration, the pin 2052 can extend slightly beyond the distal end of the hollow tube 2054, such that sharpened portions of the hollow tube 2054 can be shielded from undesired contact with objects and/or users. Portions of the pin 2052 and/or hollow tube 2054 can optionally be provided with a coating or surface treatment to reduce friction between them and/or between either component or biological tissue.

As described herein, a plurality of microneedles (e.g., microneedle 2050) can form a microneedle array 2006. FIG. 6C shows a top view of an exemplary microneedle array 2006, according to configurations of the present disclosure. In some configurations, the microneedle array 2006 can be substantially circular. As previously described herein, the microneedle array 2006 can be formed by assembling a plurality of rows of needles, either in horizontal or vertical rows. This design can be modular, and the configuration can take on any shape or size using various size rows as modules. In some configurations, all of the microneedles can be actuated, e.g., inserted into the tissue, simultaneously. In other configurations, groups or sections can be actuated sequentially. For example, the microneedle array 2006 can be divided into quadrants and each quadrant can be sequentially actuated. Sequentially can refer to actuating each row in a linear order, (e.g., row1, row2, row3), or non-linear (e.g. row1, row10, row3). Or, each row of microneedles can be separately and sequentially actuated. Additionally, each single microneedle can be separately and sequentially actuated. In some configurations, one row can be actuated at a time, e.g., 20 rows can be individually actuated in sequence, while in other configurations, two, three, four or more rows can be actuated at a time. An advantage to sequentially actuating segments of the microneedle array 2006 is that insertion of a segment can require less force on the donor site than insertion of the entire microneedle array 2006. In some configurations, the microneedle array 2006 can be driven using an actuator (e.g., a solenoid). Multiple actuations using the actuator can sequence the insertion row by row. As will be described in greater detail, the lockdown latch assembly 1126 can lock each row of microneedles in the microneedle array 2006 in an extended position after the actuator 1052 actuates (e.g., via the plunger bar engaging the hammers 1098a, 1098b) the row of microneedles from a retracted position to the extended position.

### LOCKDOWN LATCH ASSEMBLY

As previously described, the cartridge assembly 2000 can have a needle array therein (e.g., formed by a plurality of needle plates 2020). The needle array can include rigid members (e.g., arms 2022) protruding horizontally inward (see, e.g., FIG. 5C). As the microneedles are pushed into the skin, the arms 2022 can push and/or slide past latches 3006, 3008 on the lockdown latch assembly 1126 (see, e.g., FIG. 4F). The latches 3006, 3008 can be configured to secure the arms 2022 below the latches 3006, 3008, and hence also secure the microneedles (e.g., the needle plates 2020 within the cartridge assembly 2000) after deployment. As will be described herein, securing the microneedles can be accomplished via various lockdown latch assembly configurations. In various configurations, the latch(es) 3006, 3008 can permit the arms 2022 to bypass the latch(es) 3006, 3008 during extension of the microneedles (e.g., during needle deployment into a harvest site). Additionally, the latch (es) 3006, 3008 can inhibit the arms 2022 from bypassing the latch (es) 3006, 3008 after the extension of the microneedles (i.e., preventing retraction from the harvest site).

When harvesting tissue with a large needle array (i.e., an array formed of a variety of needle plates 2020 each with respective pluralities of needles), simultaneous deployment of all microneedles may be difficult. This occurs, in part, because an increase in force is used to compensate for the larger surface area of tissue. Accordingly, in some configurations, the microneedles can be deployed into the tissue in smaller quantities. This can facilitate penetration of the needle to the desired depth for tissue harvesting, as an example.

In some cases (e.g., during harvest), the elasticity of the tissue can cause the microneedles to bounce or otherwise migrate out of the tissue during needle deployment. Movement of the microneedles can disrupt the harvested tissue columns (e.g., before they can be wholly extracted). Accordingly, securing deployed microneedles can help ensure the effectiveness and efficiency of a tissue grafting process. As will be described, the lockdown latch assembly 1126 is designed to selectively secure one or more deployed microneedles during a tissue grafting process.

In some configurations, individually securing each needle plate 2020 can provide both accurate actuation and securement within the tissue. As an example, each time the actuator 1052 is actuated, a force is applied to the cartridge assembly 2000. The force can be large enough to cause impact to the needle plates 2020 that are already within the tissue (i.e., that were previously actuated). The lockdown latch assembly 1126 can be configured to lock the actuated needle plates 2020 in an extended position, ensuring that the microneedles do not withdraw or otherwise move from the tissue. Locking each needle plate 2020 enables the harvesting process to continue, while maintaining the tissue cores within the microneedles on the locked needle plate 2020. Notably, the time needed for the tissue grafting process dramatically decreases when multiple needle plates 2020 can be actuated prior to withdrawing the needles.

Furthermore, as will be described, the systems and method provided herein advantageously and synergistically operate to increase efficiency of the medical processes, while protecting sterility of the donor site, the cartridge assembly 2000 and associated components (including the needles), and the harvested tissue. That is, as will be described, a latch assembly 1126 or locking system is provided that can be automatically actuated/engaged without manual intervention and can be disposed at a location that even prevents any manual interaction with latch assembly 1126 and associated components.

As shown by FIG. 4F, the latch assembly 1126 includes a body 3002 that can be coupled to a base plate 3004. The lockdown latch assembly 1126 can further include a first latch 3006 and a second latch 3008. The components of the latch assembly 1126 are integrated with the components that are enclosed in the cartridge housing 2002, which inhibits manual or other interaction with the latch assembly 1126 or function of the latch assembly 1126 and protects the components that interact with the donor site and/or the tissue samples or in close proximity to the components that interact with the donor site and/or the tissue samples from manual or other interaction.

The first latch 3006 is positioned opposite the second latch 3008. The first latch 3006 and the second latch 3008 may be moveably coupled (e.g., slidably or pivotally) to the base plate 3004 and/or the body 3002. A spring 3010 is arranged between the first and second latches 3006, 3008. As will be described in greater detail below, the first and second latches 3006, 3008 can be selectively actuated between a plurality of positions (e.g., by the actuator 1052, Fig. 4E). The plurality of positions can include a latched position and an unlatched position. In some configurations, the latched position and the unlatched position can represent the outer bounds or limits of the plurality of positions. When the needle plate 2020 (see, e.g., FIG. 5C) is actuated from the retracted position to the extended position, the first and second latches 3006, 3008 can engage the arms 2022 on the needle plate 2020 when the first and second latches 3006, 3008 are in the latched position. The engagement of the first and second latches 3006, 3008 can prevent the needle plate 2020 from retraction (e.g., during a harvest process).

According to some configurations, the first and second latches 3006, 3008 can be fixed (e.g., non-movable inward or outward relative to the body 3002) such that the contact between the arms 2022 and the latches can cause the pair of arms 2022 to deflect outwardly until a gap between the pair of arms 2022 is sufficient to allow the needle plate 2020 to continue to move past the latches 3006, 3008 into an extended position. After the needle plate 2020 moves past the latches 3006, 3008, the pair of arms 2022 spring back inwardly.

Thus, the lockdown latch assembly 1126 can be configured to automatically lock down each needle plate 2020 during the harvest process. The user does not need to interact manually with the components of the lockdown latch assembly 1126, which is contained within the housing 1036. Once the cartridge assembly 2000 is inserted into the handheld device 1000, the lockdown latch assembly 1126 can automatically and selectively engage with the various needle plates 2020. By reducing and preventing user interaction with the lockdown latch assembly 1126 and needle plates 2020, sterility of the handheld device 1000 and cartridge assembly2000 can be maintained.

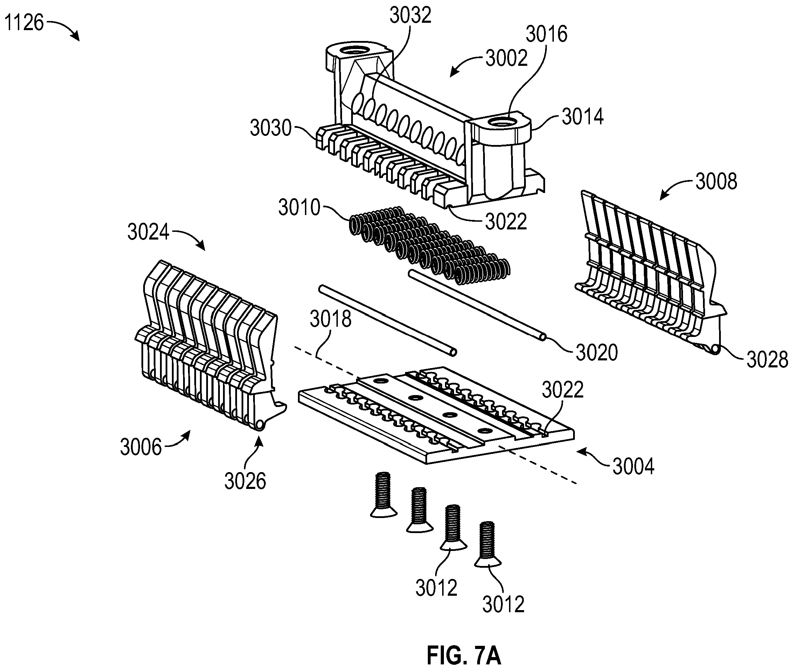

Referring now to FIGS. 7A-7C, one particular implementation of the lockdown latch assembly 1126 is shown. The lockdown latch assembly 1126 can include body 3002, base plate 3004, first latch 3006, second latch 3008, and the at least one spring 3010. The body 3002 can be removably coupled to the base plate 3004 with one or more fasteners 3012. The body 3002 can also be rigidly coupled to the needle retract slide 1110 (see, e.g., FIG. 4E) via guideposts 1124a, 1124b on opposing portions of the needle retract slide 1110. Tabs 3014 on the body 3002 can include a guidepost aperture 3016 dimensioned to receive the guideposts 1124a, 1124b such that the guideposts 1124a, 1124b can be coupled to the body 3002. The vertical carriage body 1113 can be slidably coupled to the lockdown latch assembly 1126. The vertical carriage body 1113 can include apertures 1125 configured to slidably receive the guideposts 1124a, 1124b therein such that the body 3002, and thus the lockdown latch assembly 1126, can slide or move (e.g., up or down from the perspective of Fig. 4E) with respect to the vertical carriage body 1113.

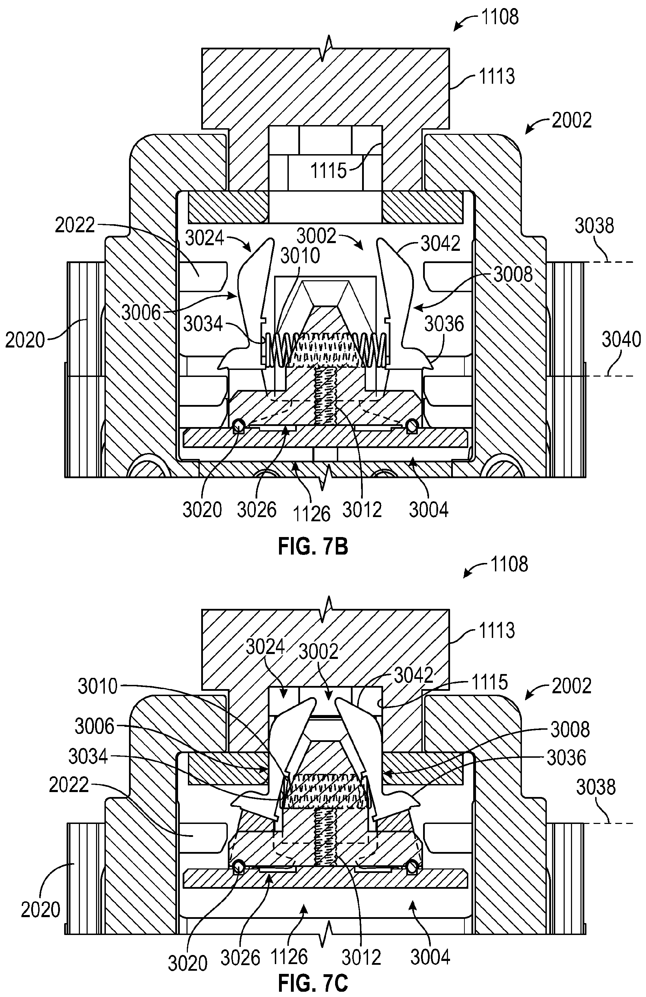

As shown, the lockdown latch assembly 1126 can include a plurality of first latches 3006 and a corresponding plurality of second latches 3008. In some configurations, the first and second latches 3006, 3008 can be arranged in complementary pairs on opposing sides of the lockdown latch assembly 1126 (see, e.g., FIG. 7B). In some configurations, the first latches 3006 and the second latches 3008 can be symmetrically placed about a longitudinal axis 3018 defined by a length of the base plate 3004 (see, e.g., FIG. 7A). In some configurations, the first and second latches 3006, 3008 can be pivotally coupled to the body 3002 and the base plate 3004 by one or more pivot pins 3020. As shown, the pivot pins 3020 can be secured between the body 3002 and the base plate 3004 in channels 3022 formed therein.

The first and second latches 3006, 3008 can be, respectively, a generally elongated member including a first end 3024 (e.g., an "upper" end from the perspective of FIG. 7A) and a second end 3026 (e.g., a "lower" end from the perspective of FIG. 7A). The first and second latches 3006, 3008 can pivot about the second end 3026 via a pin aperture 3028 dimensioned to receive the pivot pin 3020. According to some configurations, the second end 3026 of the first and second latches 3006, 3008 can be received within the body 3002 via slots 3030, which can extend laterally along a portion (e.g., from the perspective of FIG. 7A) of the body 3002.

Still referring to FIGS. 7A-7C, a spring 3010 can be arranged between each complementary pair of first and second latches 3006, 3008. In some configurations, the spring 3010 can be a coil spring that can be retained within the body 3002 via spring apertures 3032. As shown, the spring apertures 3032 can extend laterally through the body 3002, and can be dimensioned to receive the spring 3010.

With specific reference towards FIGS. 4E and 7B-7C, with the cartridge assembly 2000 installed onto the vertical component assembly 1046 (e.g., installed onto the vertical carriage assembly 1108 and locked into place via the carriage latch 1114), the vertical component assembly 1046 can be operable between a predefined "harvest" configuration (FIG. 7B) where the lockdown latch assembly 1126 is in the latched position, and a predefined "scatter" configuration (FIG. 7C) where the lockdown latch assembly 1126 is in the unlatched position. It is to be understood that numerous components of the vertical component assembly are not explicitly shown in FIGS. 7B-7C.

With the vertical component assembly 1046 in the harvest configuration (see, e.g., FIG. 7B), the spring 3010 can be configured to bias the first and second latches 3006, 3008 in the latched position (e.g., with the latches outwardly biased). In the illustrated configuration, ends of the spring 3010 can be in contact with an inside surface 3034 of the first and second latches 3006, 3008. When installed into the lockdown latch assembly 1126, the spring 3010 can be pre-biased (e.g., compressed) such that the first and second latches 3006, 3008 are biased towards the latched position (see, e.g., FIG. 7B).

As shown, the first and second latches 3006, 3008 can have a protrusion 3036 extending horizontally outward therefrom. In some configurations, the protrusion 3036 can be arranged between the first end 3024 and the second end 3026. During deployment of the needle plates 2020 from the retracted position 3038 to the extended position 3040 (e.g., via the actuator 1052 driving the plunger bar 1106 into the hammers 1098a, 1098b), the inwardly extending arms 2022 (i.e., rigid members) are moved downward and contact the protrusion 3036. The contact between the arms 2022 and the protrusion 3036 cause the first and second latches 3006, 3008 to pivot inwardly, thereby compressing the spring 3010. The pivoting of the first and second latches 3006, 3008 allow the needle plate 2020 to continue to move past the protrusions 3036 and into the extended position 3040. After the needle plate 2020 moves past the protrusions 3036, the first and second latches 3006, 3008 can move back into the latched position owing to the spring 3010.

Once the needle plate 2020 is in the extended position 3040, the protrusion 3036 on the first and second latches 3006, 3008 prevent the needle plate 2020 from inadvertently returning to the retracted position 3038 (see, e.g., FIG. 7B). For example, if an outside force were to act on the needle plate 2020 in an upwards direction, the protrusion 3036 would engage a top side of the arm 2022, thereby holding the needle plate 2020 in the extended position 3040. As such, when the vertical component assembly is in the harvest configuration, the lockdown latch assembly 1126 can be configured to allow the needle plate 2020 to be deployed from the retracted position 3038 to the extended position 3040, but prevent or occlude the needle plate 2020 from retracting.

During the transition from the harvest configuration to the scatter configuration, the lockdown latch assembly 1126 can move upwards (e.g., along guideposts 1124a, 1124b) towards the vertical carriage body 1113 of the vertical carriage assembly 1108. As the lockdown latch assembly 1126 moves upwards, the base plate 3004 can engage and apply force to a bottom side of the arms 2022 of the needle plate 2020, thereby retracting the needle plate 2020. Additionally, during the upward motion of the lockdown latch assembly 1126, an outside surface 3042 of the first and second latches 3006, 3008 can contact the sides of a recess 1115 formed in the vertical carriage body 1113 (see, e.g., FIG. 7C).

The contact between the first and second latches 3006, 3008 and the sides of the recess 1115 can cause the first and second latches 3006, 3008 to pivot inwardly into the unlatched position (see, e.g., FIG. 7C), thereby compressing the spring 3010. In some situations, the pivoting of the first and second latches 3006, 3008 into the unlatched position can prevent the needle plate 2020 from occlusion during retraction of the needle plate. For example, with the first and second latches 3006, 3008 in the unlatched position, the protrusion 3036 is removed from the pathway of the arm 2022, thereby allowing uninhibited retraction of the needle plate 2020. The movement of the first and second latches 3006, 3008 into the unlatched position also allows for a more efficient packaging of the lockdown latch assembly 1126 when in the scatter configuration.

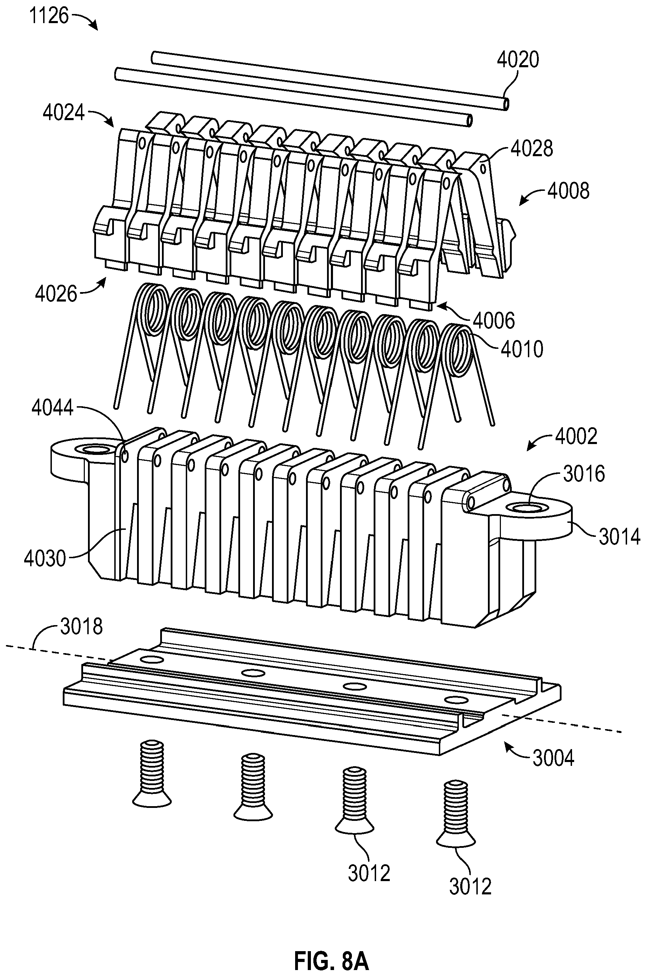

Referring now to FIGS. 8A-8C, another implementation of the lockdown latch assembly 1126 is shown. In the following illustrations, like elements will be referenced using like numerals. Notably, the implementation of the lockdown latch assembly 1126 shown in FIGS. 8A-8C includes a pin aperture 4044 through which the first and second latches 4006, 4008 are coupled to a body 4002 about a first end 4024, as opposed to a second end 4026. Other aspects between the embodiments that are the same or substantially similar will not be repeated. As such, it is to be understood that, unless stated or shown otherwise, elements reference with like numerals can function the same or substantially similarly to those of the other embodiments.

In the illustrated configuration, the first and second latches 4006, 4008 can be pivotally coupled to a body 4002 by one or more pivot pins 4020. In some configurations, the body 4002 can include the pin aperture 4044 dimensioned to receive the pivot pin 4020 therein. In some configurations, end walls can be coupled to laterally opposing ends (i.e., left or right sides from the perspective of Fig. 8A) of either one of the body 4002 or the base plate 3004, or be integral to the base plate 3004. For example, end walls, if integral to the base plate 3004, can extend vertically upwards from the base plate 3004. In the illustrated configuration, the end walls can include the tabs 3014 extending outwardly therefrom. The end walls can serve to block the pin apertures 4044 on the body 4002 to prevent the pivot pins 4020 from inadvertent removal once the pivot pins 4020 are installed. In the illustrated configuration, the first and second latches 4006, 4008 can pivot about the first end 4024 via a pin aperture 4028 dimensioned to receive the pivot pin 4020 therein. In the illustrated configuration, the first and second latches 4006, 4008 can be received within the body 4002 via slots 4030 extending horizontally inward from opposing lateral sides (e.g., see, e.g., FIG. 8A) of the body 4002. In the illustrated configuration, a spring 4010 can be arranged between each pair of first and second latches 4006, 4008. In some configurations, for example, the spring can be a torsion spring.

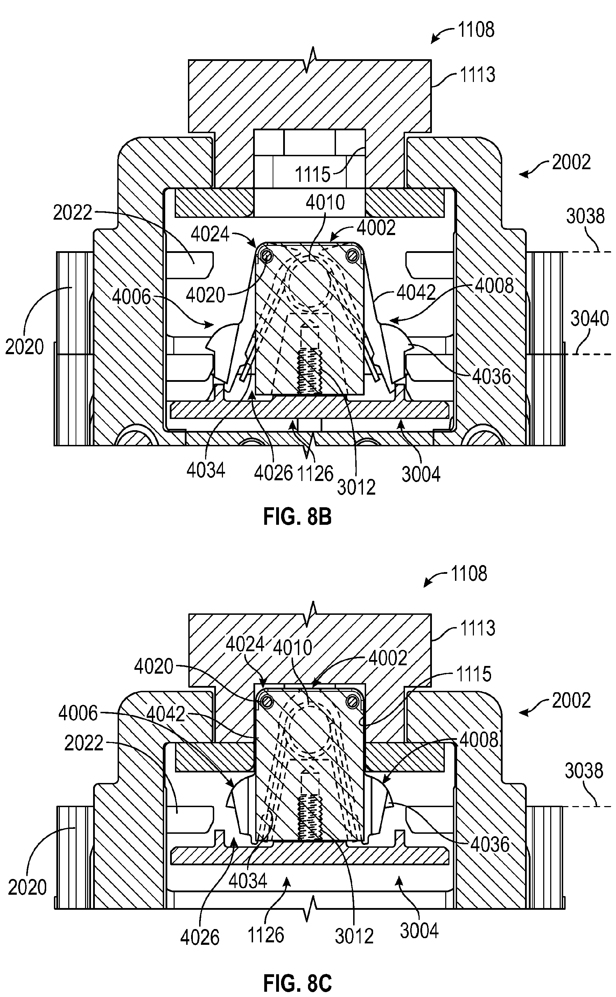

With the vertical component assembly 1046 in the harvest configuration (see, e.g., FIG. 8B), the spring 4010 can be configured to bias the first and second latches 4006, 4008 in the latched position (e.g., with the latches outwardly biased). In the illustrated configuration, ends of the spring 4010 can be in contact with an inside surface 4034 of the first and second latches 4006, 4008. When installed into the lockdown latch assembly 1126, the spring 4010 can be pre-biased (e.g., compressed) such that the first and second latches 4006, 4008 are biased towards the latched position (see, e.g., FIG. 8B). In the illustrated configuration, the spring 4010 can include legs extending from a coil portion of the spring. In some configurations, the legs can include a bend. In some configurations, a rod can extend between end walls of the body 4002 and through the coil portion of the spring 4010. In that way, the rod can retain the positioning of the spring 4010 relative to the body 4002.

As shown, the first and second latches 4006, 4008 can have a protrusion 4036 extending horizontally outward therefrom. In some configurations, the protrusion 4036 can be arranged between the first end 4024 and the second end 4026. In the illustrated configuration, the protrusion 4036 can define a width (i.e., into and out of the page from the perspective of Fig. 8B) that spans at least a portion of the width of the first or second latches 4006, 4008. In some configurations, the protrusion 4036 can define a width that spans the entire width of the first or second latches 4006, 4008. In yet further configurations, the protrusion 4036 can define a width that spans beyond the width of the first or second latches 4006, 4008.

During deployment of the needle plates 2020 from the retracted position 3038 to the extended position 3040 (e.g., via the actuator 1052 driving the plunger bar 1106 into the hammers 1098a, 1098b), the inwardly extending arms 2022 (i.e., rigid members) are moved downward and contact the protrusion 4036. The contact between the arms 2022 and the protrusion 4036 cause the first and second latches 4006, 4008 to pivot inwardly, thereby compressing the spring 4010. The pivoting of the first and second latches 4006, 4008 allow the needle plate 2020 to continue to move past the protrusions 4036 and into the extended position 3040. After the needle plate 2020 moves past the protrusions 4036, the first and second latches 4006, 4008 can spring back into the latched position owing to the spring 4010.

Once the needle plate 2020 is in the extended position 3040, the protrusion 4036 on the first and second latches 4006, 4008 prevents the needle plate 2020 from inadvertently returning to the retracted position 3038 (see, e.g., FIG. 8B). For example, if an outside force were to act on the needle plate 2020 in an upwards direction, the protrusion 4036 would engage a top side of the arm 2022, thereby holding the needle plate 2020 in the extended position 3040. As such, when the vertical component assembly is in the harvest configuration, the lockdown latch assembly 1126 can be configured to allow the needle plate 2020 to be deployed from the retracted position 3038 to the extended position 3040, but prevent or occlude the needle plate 2020 from retracting.

During the transition from the harvest configuration to the scatter configuration, the lockdown latch assembly 1126 can move upwards (e.g., along guideposts 1124a, 1124b) towards the vertical carriage body 1113 of the vertical carriage assembly 1108. As the lockdown latch assembly 1126 moves upwards, the base plate 3004 can engage and apply force to a bottom side of the arms 2022 of the needle plate 2020, thereby retracting the needle plate 2020. Additionally, during the upward motion of the lockdown latch assembly 1126, an outside surface 4042 of the first and second latches 4006, 4008 can contact the sides of a recess 1115 formed in the vertical carriage body 1113 (see, e.g., FIG. 8C).

The contact between the first and second latches 4006, 4008 and the sides of the recess 1115 can cause the first and second latches 4006, 4008 to pivot inwardly into the unlatched position (see, e.g., FIG. 8C), thereby compressing the spring 4010. In addition to the other benefits previously described herein, the pivoting of the first and second latches 4006, 4008 into the unlatched position can prevent the needle plate 2020 from occlusion during retraction of the needle plate. For example, with the first and second latches 4006, 4008 in the unlatched position, the protrusion 4036 is removed from the pathway of the arm 2022, thereby allowing uninhibited retraction of the needle plate 2020.

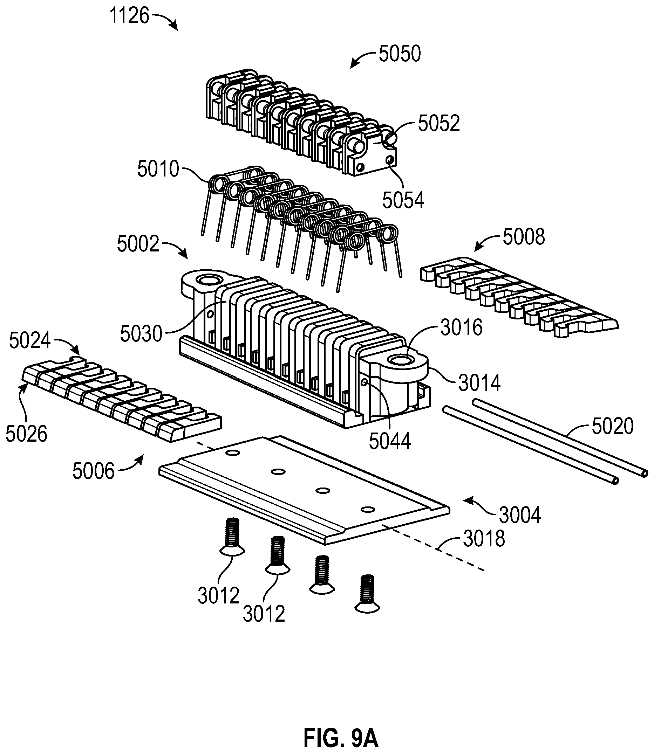

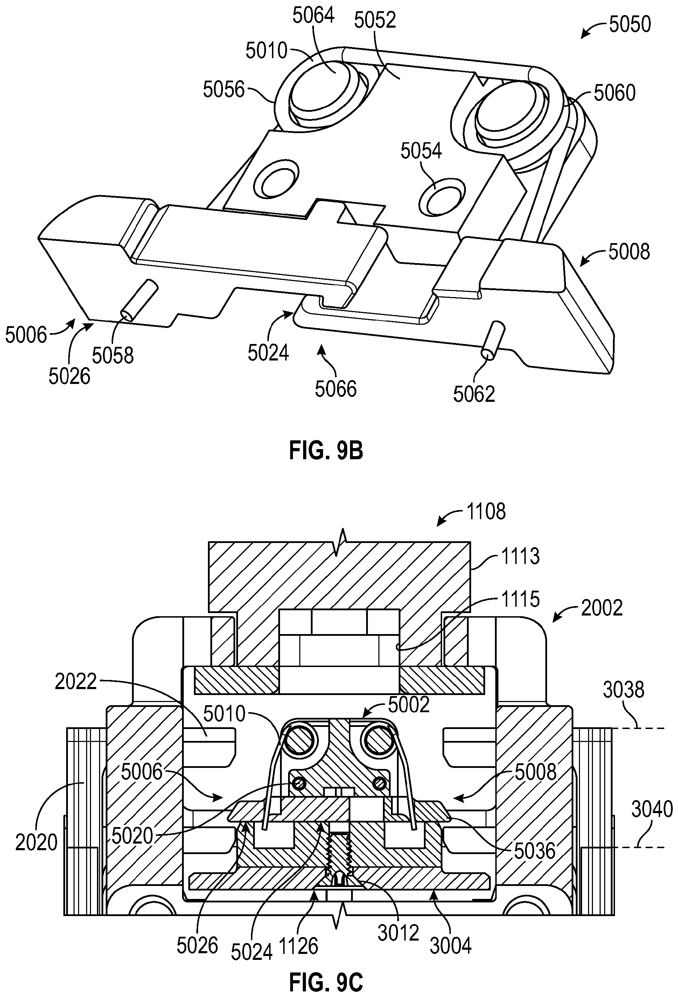

Referring now to FIGS. 9A-9D, another configuration of the lockdown latch assembly 1126 is shown. In the following illustrations, like elements will be referenced using like numerals. Notably, the implementation of the lockdown latch assembly 1126 shown in FIGS. 9A-9D includes horizontally opposed first and second latches 5006, 5008 that can be slidably coupled to a body 5002. In the illustrated configuration, the first and second latches 5006, 5008 can slide horizontally outward and inward (e.g., with respect to the body 5002) between the latched position and the unlatched position. Other aspects between the embodiments that are the same or substantially similar will not be repeated. As such, it is to be understood that, unless stated or shown otherwise, elements reference with like numerals can function the same or substantially similarly to those of the other embodiments.

In the illustrated configuration, the first and second latches 5006, 5008 can be integrated into a latch assembly 5050. The latch assembly 5050 can be coupled to the body 5002 by one or more pins 5020. In the illustrated configuration, the latch assembly 5050 can include a vertical plate 5052. The vertical plate can include a pin aperture 5054 dimensioned to receive the pin 5020 therein, thus allowing the latch assembly 5050 to be secured to the body 5002 via the pins 5020. The latch assembly 5050 can be received within the body 5002 via slots 5030 extending horizontally inward from opposing lateral sides (e.g., from the perspective of FIG. 9A) of the body 5002.

In the illustrated configuration, a spring 5010 can be arranged between each pair of first and second latches 5006, 5008. In some configurations, the spring 5010 can be a double torsion spring, including a first coil portion 5056 with a first end 5058 extending therefrom, and a second coil portion 5060 with a second end 5062 extending therefrom. The vertical plate 5052 can include a cylindrical protrusion 5064 that can extend through the first and/or second coil portions 5056, 5060, thereby securing the spring 5010 to the vertical plate 5052. In addition, the first end 5058 of the spring 5010 can be coupled to the first latch 5006 and the second end 5062 of the spring 5010 can be coupled to the second latch 5008.

In the illustrated configuration, the first and second latches 5006, 5008 can slide horizontally inward and outward along a bottom edge of the vertical plate 5052. In some configurations, the first and second latches 5006, 5008 can include an interlocking portion 5066 arranged at the first end 5024. The interlocking portion 5066 can be configured to enable the first ends 5024 of the first and second latches 5006, 5008 to slide past or alongside each other within the slot 5030 formed in the body 5002. In some configurations, the interlocking portion 5066 may define the outward most position of the first and second latches 5006, 5008 (e.g., the latched position).

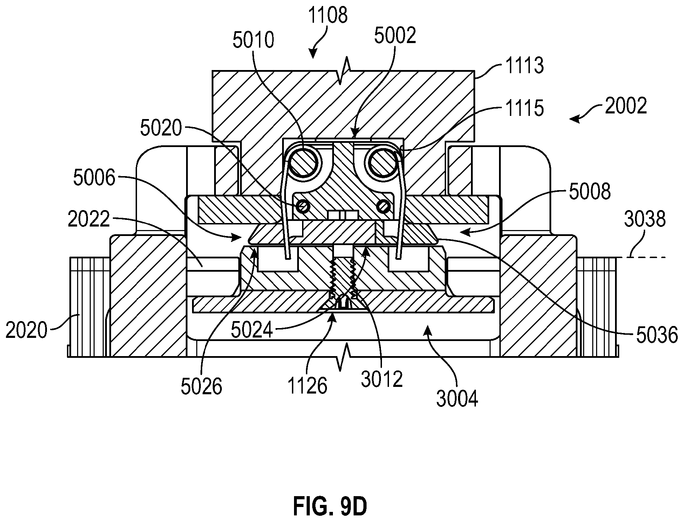

With specific reference towards FIGS. 4E and 9C-9D, with the cartridge assembly 2000 installed onto the vertical component assembly 1046 (e.g., installed onto the vertical carriage assembly 1108 and locked into place via the carriage latch 1114), the vertical component assembly 1046 can be operable between the "harvest" configuration (FIG. 9C) where the lockdown latch assembly 1126 is in a latched position, and the "scatter" configuration (FIG. 9D) where the lockdown latch assembly 1126 is in an unlatched position.

In the illustrated harvest configuration (see, e.g., FIG. 9C), the protrusion 5036 can be arranged at the second ends 5026 of the first and second latches 5006, 5008. During deployment of the needle plates 2020 from the retracted position 3038 to the extended position 3040, the inwardly extending arms 2022 (i.e., rigid members) are moved downward and contact the protrusion 5036. The contact between the arms 2022 and the protrusion 5036 can cause the first and second latches 5006, 5008 to slide or move inwardly, thereby compressing the spring 5010 and allowing the needle plate to continue to move past the protrusions 5036 and into the extended position 3040. After the needle plate 2020 moves past the protrusions 5036, the first and second latches 5006, 5008 spring back into the latched position owing to the spring 5010.

As the lockdown latch assembly 1126 moves upwards to the scatter configuration (see, e.g., FIG. 9D), the arms of the spring 5010 (e.g., the portion of the spring between the coil portion and the ends) can contact the sides of a recess 1115 formed in the vertical carriage body 1113. This contact between the arms of the spring and the sides of the recess 1115 can cause the first and second latches 5006, 5008 to slide or move inwardly into the unlatched position (e.g., via the coupling between the first and second latches 5006, 5008 and the first and second ends 5058, 5062 of the spring 5010).

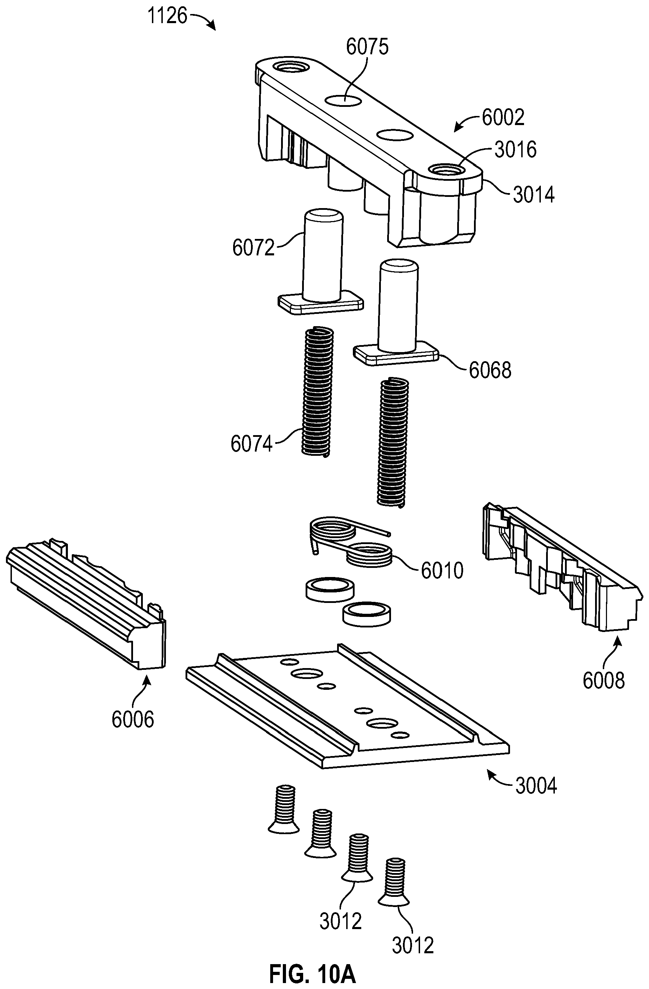

Referring now to FIGS. 10A-10D, another configuration of the lockdown latch assembly 1126 is shown. In the following illustrations, like elements will be referenced using like numerals. Notably, the implementation of the lockdown latch assembly 1126 shown in FIGS. 10A-10D includes horizontally opposed first and second latches 6006, 6008 that can be slidably coupled to a body 6002 and moved between the latched and unlatched positions by the positioning of a guide plate 6068 along a guide profile 6070 formed into the first and second latches 6006, 6008. In the illustrated configuration, the first and second latches 6006, 6008 can slide horizontally outward and inward (e.g., with respect to the body 6002) between the latched position and the unlatched position. Other aspects between the embodiments that are the same or substantially similar will not be repeated. As such, it is to be understood that, unless stated or shown otherwise, elements reference with like numerals can function the same or substantially similarly to those of the other embodiments.

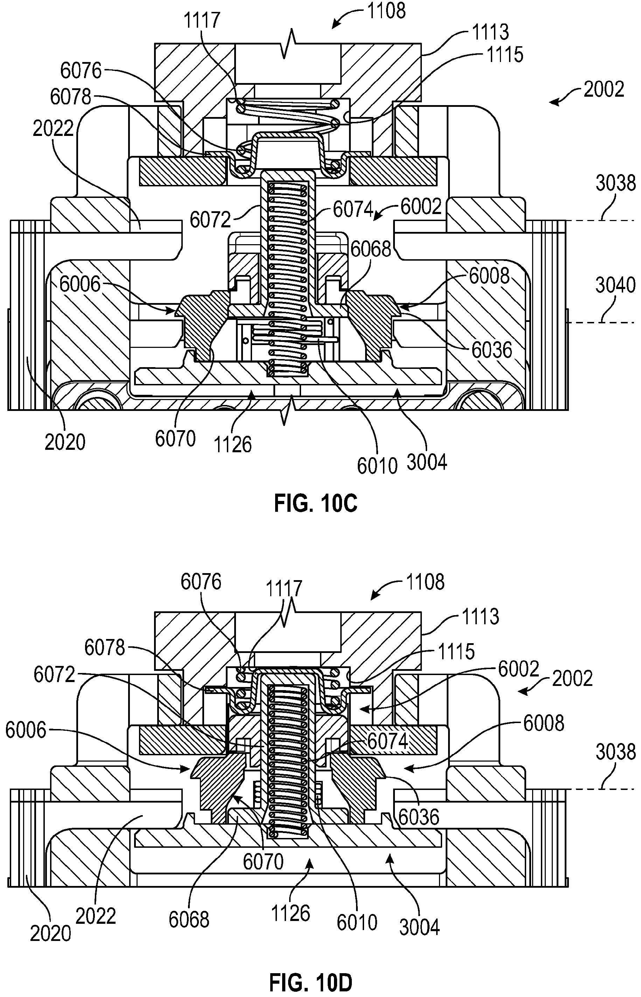

With specific reference towards FIGS. 4E and 10A-10D, with the cartridge assembly 2000 installed onto the vertical component assembly 1046 (e.g., installed onto the vertical carriage assembly 1108 and locked into place via the carriage latch 1114), the vertical component assembly 1046 can be operable between the "harvest" configuration (FIG. 10C) where the lockdown latch assembly 1126 is in a latched position, and the "scatter" configuration (FIG. 10D) where the lockdown latch assembly 1126 is in an unlatched position.

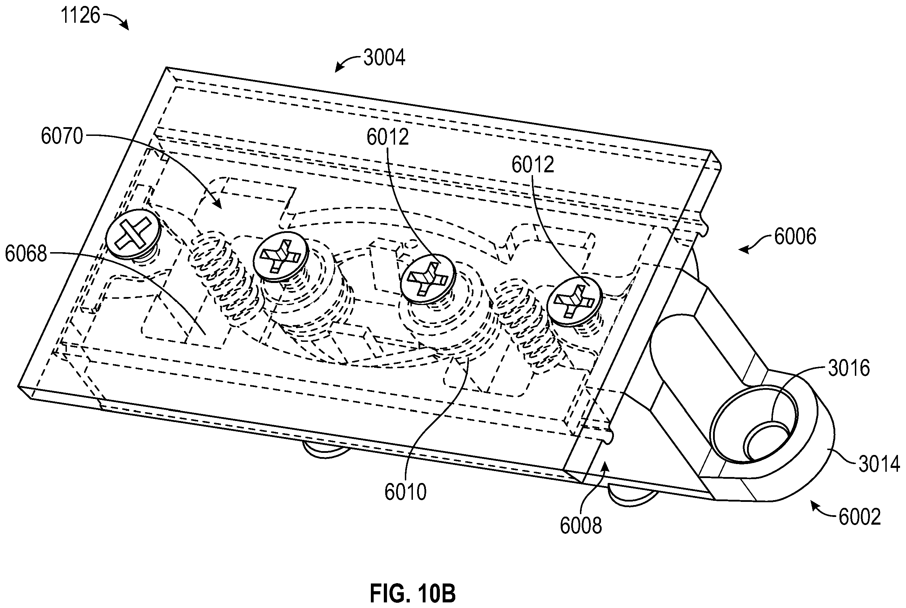

With the vertical component assembly 1046 in the harvest configuration, the spring 6010 can be configured to bias the first and second latches 6006, 6008 towards the unlatched position (e.g., with the latches inwardly biased). In the illustrated configuration, ends of the spring 6010 can be in contact with a post (see, e.g., FIG. 10B) protruding from an interior of the first and second latches 6006, 6008. When installed into the lockdown latch assembly 1126, the springs 6010 can be pre-biased (e.g., compressed) such that the first and second latches 6006, 6008 are biased towards the unlatched position.

In the illustrated configuration, the guide plate 6068 can have a cylindrical shaft 6072 coupled thereto. The shaft 6072 can be slidably coupled to the body 6002 and received in shaft apertures 6075 formed therein. In some configurations, a coil spring 6074 can be received within an internal bore of the shaft 6072. When the lockdown latch assembly 1126 is in the latched position, the coil spring 6074 can be configured to bias the guide plate 6068 upwards, thereby holding the first and second latches 6006, 6008 in the latched position owing to the sloped shape of the guide profile 6070.

As shown, the first and second latches 6006, 6008 can have a protrusion 6036 extending horizontally outward therefrom. During deployment of the needle plates 2020 from the retracted position 3038 to the extended position 3040, the inwardly extending arms 2022 (i.e., rigid members) are moved downward and contact the protrusions 6036. The contact between the arms 2022 and the protrusion 6036 can cause the pair of arms 2022 to deflect outwardly until a gap between the pair of arms 2022 is sufficient to allow the needle plate 2020 to continue to move past the protrusions 6036 into the extended position 3040 (see, e.g., FIG. 10C). After the needle plate 2020 moves past the protrusions 6036, the pair of arms 2022 spring back inwardly. As such, when the lockdown latch assembly 1126 is in the latched configuration, the first and second latches 6006, 6008 are prevented or inhibited from moving or sliding horizontally inward due to the contact between the guide plate 6068 and the guide profile 6070.

During the transition from the harvest configuration to the scatter configuration (see, e.g., FIG. 10D), the lockdown latch assembly 1126 can move upwards towards the vertical carriage body 1113 of the vertical carriage assembly 1108. As the lockdown latch assembly 1126 moves upwards, the shaft 6072 can contact a flange 1117 formed in the recess 1115. The contact between the flange 1117 and the shaft 6072 causes the coil spring 6074 to compress, thereby driving the shaft 6072, and thus the guide plate 6068, downwards relative to the body 6002. As the guide plate 6068 moves downwards, the first and second latches 6006, 6008 begin to move or slide horizontally inward due to the spring 6010 biasing the first and second latches 6006, 6008 towards the unlatched position, and the sloped shape of the guide profile 6070.

In some configurations, a second coil spring 6076 and a spring cup 6078 can be arranged between an upper distal end of the shaft 6071 and the flange 1117 within the recess 1115. The second coil spring 6076 can have a higher spring force than that of the coil spring 6074. In this configuration, when transitioning from the harvest configuration to the scatter configuration, the weaker coil spring 6074 can compress first, followed by the stronger second coil spring 6076. This can, for example, prevent the lockdown latch assembly 1126 from changing between the latched/unlatched positions during carriage locking.

Various other latch and spring configurations are also envisioned. For example, a latch (e.g., any one of latches 3006, 3008, 4006, 4008, 5006, 5008, 6006, 6008) can have a spring integrally formed into the latch. In such a configuration, the latch can be designed with a thin, spring-like protrusion extending from a body of the latch (e.g., similar to that of a leaf spring). For example, the thin protrusion may extend out from the latch to be in contact with a body (e.g., any one of bodies 3002, 4002, 5002, 6002) to bias the latch in a latched position. In some configurations, the thin protrusion may be shaped like an arc. In other configurations, the thin protrusion may extend out from the latch to be in contact with a base plate (e.g., any of base plate 3004) to bias the latch in a latched position.

Various other body and base plate configurations are also envisioned. For example, a body (e.g., any one of bodies 3002, 4002, 5002, 6002) and a base plate (e.g., base plate 3004) could be formed as a unitary component. For example, the base plate and the body can be combined to form a single piece body with an integrated base plate. In addition, in some configurations the body can be modular. For example, the body can be split into a plurality of sections, where each section can be configured to receive one or more pairs of latches. The sections can be modularly coupled together to form a complete body. The modularity of the body can provide the benefit of making the parts easier to manufacture. Further, the end walls that can be part of the base plate may act to prevent the pivot pins 4020 from sliding out.

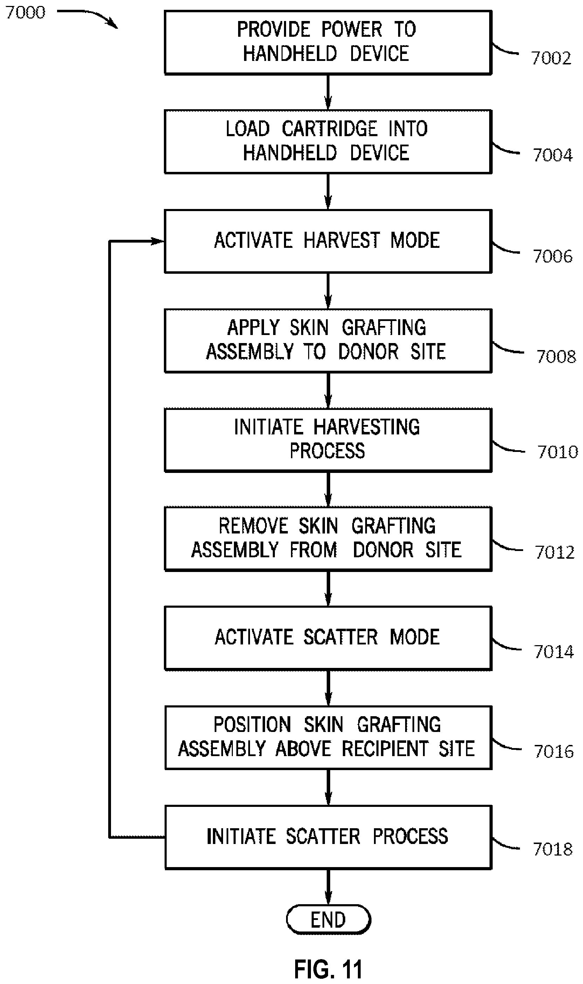

Referring now to FIG. 11, some non-limiting examples of steps of a process 7000 for harvesting and scattering tissue is shown, according to configurations of the present disclosure. In some configurations, the process 7000 can be implemented using the skin grafting system 100, as described above. As shown, the process 7000 includes providing power to the handheld device (process block 7002). In some configurations, the handheld device can be the same or similar to handheld device 1000. The process 7000 is shown to further include loading a cartridge into the handheld device (process block 7004). In some configurations, the cartridge can be the same or similar to the cartridge assembly 2000. Further, the process 7000 is shown to include activating a harvest mode (process block 7006). This activation can be initiated via user interface 1008, according to some configurations, such as will be described. Alternatively, the activation can be initiated via contact with a donor site. The process 7000 is shown to include applying a skin grafting system (e.g., skin grafting system 100) to a donor site (process block 7008). The donor site can correspond to a healthy area of tissue on a patient. Next, the process 7000 is shown to include initiating a harvesting process (process block 7010). In some configurations, this initiation can occur via the above-described trigger 1014. The process 7000 is shown to further include removing the skin grafting system from the donor site (process block 7012). Next, the process 7000 is shown to include activating a scatter mode (process block 7014). In some configurations, this activation can occur via user interface 1008, such as will be described. The process 7000 is shown to further include positioning the skin grafting system above a recipient site (process block 7016). In some configurations, the recipient site can correspond to a damaged area of tissue on the patient. Next, the process 7000 is shown to include initiating a scatter process (process block 7018). In some configurations, this initiation can occur via actuation of the above-described trigger 1014. As shown, the process 7000 can end after the scatter process (process block 7018), or can return to process block 7006 to reactivate the harvest mode. In some configurations, a single cartridge (e.g., cartridge housing 2002) can be used multiple times on the same patient. Advantageously, if the recipient site is relatively large, multiple harvests and scatters can occur using a single cartridge. Accordingly, the process 7000 can continue with process blocks 7006 through 7018 until a user is ready to dispose of the cartridge.

According to configurations of the present disclosure, the harvest process and scatter process can be performed using skin grafting system 100. A non-limiting description of the internal functions of the handheld device 1000 and cartridge assembly 2000 are accordingly disclosed herein.

### USER INTERFACE

Referring to FIG. 2B, as one non-limiting example, an example of using the user interface 1008 to control the above-described process is provided. Upon providing power to the handheld device, the stand-by input 1018 can flash green when the handheld device 1000 first powers on (e.g., for ~8 seconds at initial start-up). This can inform the user that the handheld device 1000 is performing a start-up self-test or other operation. As another non-limiting example, the stand-by input 1018 can produce steady green illumination when the handheld device 1000 is on and ready for subsequent use. In some configurations, pressing the stand-by input 1018 for a pre-determined amount of time (e.g., 3 seconds, 5 seconds, or the like) can cause the handheld device 1000 to enter a stand-by mode. Continuing with the non-limiting example, the stand-by input 1018 can stop producing light when the handheld device 1000 is in stand-by mode. Other light colors, patterns, and timing can be implemented, according to various configurations and preferences.

As another non-limiting example, the indicator light 1020 can produce steady white light when the handheld device 1000 is in harvest mode but sufficient pressure against a donor site has not been achieved, such as will be described during a skin grafting process. Further, the indicator light 1020 can produce steady green light when the handheld device 1000 is in harvest mode and sufficient pressure against the donor site has been achieved (and the trigger 1014 is disengaged). The indicator light 1020 can produce flashing green light when the handheld device 1000 is in the process of harvesting. If pressure drops below a threshold value during the harvesting process, the indicator light 1020 can produce flashing white light. Further, the indicator light 1020 can produce flashing white light when the handheld device 1000 is experiencing a fault condition.

In another non-limiting example, the scatter input 1022 can produce steady white light when the harvest process is complete. In some configurations, a subsequent press of the scatter input 1022 can cause the handheld device 1000 to enter a scatter mode. The scatter input 1022 can produce steady green light when the handheld device 1000 is in scatter mode. Similar to the indicator light 1020, the scatter input 1022 can produce flashing white light when the handheld device 1000 is experiencing a fault condition. In some configurations, the scatter input 1022 can produce flashing white light during the harvesting process, which can indicate that extraction recovery is needed. A subsequent press of the scatter input 1022 can activate an extraction recovery process. Once the extraction recovery process is complete, the scatter input 1022 can produce a steady white light. A detailed description of the extraction recovery process is provided below.

In some configurations, similar to the indicator light 1020, the indicator light 1016 can produce a solid green light when the handheld device 1000 is in the harvest mode and sufficient pressure against the donor site has been achieved (and the trigger 1014 is disengaged). Additionally, the indicator light 1016 can produce flashing green light during the harvesting process, according to some configurations.

### SKIN GRAFTING SYSTEM OPERATING POSITIONS

In some configurations, a plurality of operating positions corresponding to the skin grafting system 100 can be defined. Notably, the skin grafting system 100 can operate using additional operating positions not explicitly defined.

Some configurations of the present disclosure include a horizontal carriage home position, where the horizontal carriage assembly 1082 can be in a position that occludes the horizontal flag sensor 1064. This position can be a "safe" position that keeps the horizontal carriage away from other moving parts.

Some configurations of the present disclosure include a vertical carriage harvest position, corresponding to a calibrated position where the vertical carriage assembly 1108 can be aligned with the corresponding components for loading or for harvesting. This position can be below the vertical flag sensor occlusion point. From a user's perspective, it can appear that the vertical carriage assembly 1108 is closest to the engagement slot 1002 of the handheld device 1000.

Some configurations of the present disclosure include a vertical carriage unlock/scatter position corresponding to a calibrated position where the vertical carriage assembly 1108 has unlocked the needle retract slide 1110 by pushing the needle retract slide latches 1116a, 1116b over their respective unlock cams 1102a, 1102b. This can be the highest position the vertical carriage assembly 1108 will travel to. From a user's perspective, it can appear that the vertical carriage assembly 1108 is up inside the handheld device 1000.

Some configurations of the present disclosure include a "flipper in" position and a "flipper out" position. Each flipper 1074 can have two defined positions that the handheld device 1000 detects via flag sensors that can provide positive feedback that each position has been reached. The "flipper in," or retracted, position can correspond to when the flipper 1074 is safely away from moving parts. The "flipper out," or extended, position can correspond to when the flipper 1074 is blocking the top plate 1112. The "flipper out" position can be used for initialization, when the needle retract slide 1110 (and therefore the cartridge assembly 2000) is locked.

Some configurations of the present disclosure include a vertical carriage lock position, corresponding to a calibrated position where the vertical carriage assembly 1108 can move (with the flippers 1074 extended out) to compress the needle retract springs 1120 between the top plate 1112 and the vertical carriage body 1113 to lock the needle retract slide latches 1116. This "locking" is what can allow the microneedles to later be retracted, while also locking the cartridge assembly 2000 inside the handheld device 1000.

Some configurations of the present disclosure include a vertical carriage lock relax position, which can be a position that is offset from a calibrated lock position, where a properly locked needle retract slide top plate 1112 will no longer be putting pressure on the flippers 1074, and therefore the flippers 1074 can be safe to retract in. Conversely, if the needle retract slide top plate 1112 is not properly locked, this position can be designed to maintain enough pressure on the flippers 1074 so that they will not retract in. This position can enable the handheld device 1000 to positively sense a proper locking of the needle retract slide 1110.

Some configurations of the present disclosure include a vertical carriage extract position, which can be a position that is offset from a calibrated unlock position, where the needle retract slide 1110 will not be unlocked and the extended microneedles can be behind the tissue stabilizer 2014. After harvest, this position is where the vertical carriage assembly 1108 can go to extract the microneedles (containing the tissue columns) from the tissue prior to scattering. Advantageously, tissue grafts may not be exposed in this position, as the microneedles remain extended.

Some configurations of the present disclosure include a harvest recovery mode, which can occur during the harvest process. The harvest recovery mode can include attempting to continue deploying the needle plates into the tissue. Additionally, the harvest recovery mode can be automatic and fully controlled by on-board software (i.e., no user interaction required). In some embodiments, the harvest recovery mode can include reversing the motion of the horizontal carriage assembly 1082 by a predetermined distance or time interval. Subsequently, the horizontal carriage assembly 1082 can advance and again attempt to deploy the needle plates into the tissue.

Some configurations of the present disclosure include an extraction recovery mode, which can occur after the microneedles have been deployed (and the handheld device 1000 is attempting to return the horizontal carriage to its home position). In some configurations, it may be possible for the horizontal carriage assembly 1082 to get stuck due to increased friction from the needle plates. If this occurs, the handheld device 1000 can blink the scatter light (on the scatter input 1022) white, indicating that an extraction recovery is needed. The user may then relieve the downward force on the tissue, and press the scatter input 1022, which will allow the handheld device 1000 to continue with extracting the microneedles from the tissue.

### SKIN GRAFTING ASSEMBLY VERTICAL OPERATION

Various components corresponding to the handheld device 1000 and cartridge assembly 2000 can have a predefined operation based on the current mode of the handheld device 1000 (e.g., initialization, harvest mode, scatter mode, etc.), according to some configurations.

In some configurations, the vertical component assembly 1046 can have a predefined "loading" configuration that corresponds to loading of the cartridge assembly 2000 into the handheld device 1000. During loading, for example, the actuator plunger bar 1106, each flipper 1074, and the needle retract slide 1110 can be retracted (the microneedles retracted). The vertical carriage assembly 1108 can be set to the harvest position (as described above).

In some configurations, the vertical component assembly 1046 can have a predefined "initialization" configuration. During initialization, for example, each flipper 1074 can be extended (flipper out), and the needle retract slide 1110 can be locked with the needle retract springs 1120 loaded (the microneedles remain retracted). The vertical carriage assembly 1108 can be set to the lock position (see above). With each flipper 1074 extended, the vertical carriage assembly 1108 can move up to the lock position. The extended flippers 1074 can hold the needle retract slide 1110 in place. When the vertical carriage assembly 1108 reaches the lock position, the needle retract slide latches 1116 can lock the top plate 1112 in place with the needle retract springs 1120 loaded. In some configurations, this does not move the microneedles from their retracted state.

In some configurations, the vertical component assembly 1046 can have a predefined "initialized" configuration, which can correspond to the skin grafting system 100 being ready to harvest. During the initialized configuration, for example, each flipper 1074 can be retracted (flipper in), and the needle retract slide 1110 can be locked with the needle retract springs 1120 loaded. In some configurations, this does not move the microneedles from their retracted state. The vertical carriage assembly 1108 can move back down to the harvest position, according to some configurations.

In some configurations, the vertical component assembly 1046 can have a predefined "harvest" configuration corresponding to an applied user force. During the harvest configuration, for example, the needle retract slide 1110 can remain locked with the needle retract springs 1120 loaded and the microneedles retracted. The vertical carriage assembly 1108 can remain in the harvest position, according to some configurations. When the user positions the skin grafting system 100 at the donor site and applies downward force, the user will detect the tissue stabilizer 2014 moving a small amount in the direction opposite to the applied force, causing the indicator lights 1016 and 1020 to light up, indicating to the user that there exists proper alignment for harvest. In some configurations, the indicator light 1016 can illuminate green, to provide a visual confirmation to the user that a sufficient force has been applied.

In some configurations, the vertical component assembly 1046 can have a predefined "harvest" configuration corresponding to needle deployment. During this harvest configuration, for example, the actuator plunger bar 1106 can advance, and the needle retract slide 1110 can remain locked with the needle retract springs 1120 loaded. Notably, the microneedles (e.g., from microneedle array 2006) can be deployed into the tissue. The vertical carriage assembly 1108 can remain at the harvest position, and a user force can still be applied via the handheld device 1000, according to some configurations. When the user pulls the trigger 1014, the skin grafting system 100 can begin the harvest sequence. Accordingly, the skin grafting system 100 can advance each microneedle array row of microneedles into the tissue by hitting the hammers 1098a, 1098b with the actuator plunger bar 1106.

In some configurations, the vertical component assembly 1046 can have a predefined "extraction" configuration. During the extraction configuration, for example, the actuator plunger bar 1106 can be retracted, the needle retract slide 1110 can remain locked with the needle retract springs 1120 loaded. The microneedles (e.g., from microneedle array 2006) can remain deployed into the tissue at the start of extraction. The vertical carriage assembly 1108 can move to the extraction position (described above). In some configurations, after the harvest is complete, the skin grafting system 100 can extract the microneedles by lifting all of microneedles within the microneedle array 2006 at once. The microneedles can be lifted up to the extraction position, and the user force can be removed. In some configurations, the microneedles can remain advanced relative to the pins (e.g., pin 2052) and the tissue stabilizer 2014 can remain stationary when the microneedles are retracted.

In some configurations, the vertical component assembly 1046 can have a predefined "scatter" configuration. During the scatter configuration, for example, the needle retract slide 1110 can be in a retracted position, with the microneedles similarly retracted. In some configurations, the vertical carriage assembly 1108 can move from the extracted position. When the user activates the scatter sequence, the skin grafting system 100 can move the vertical carriage assembly 1108 from the extracted position, which can release the loaded needle retract springs 1120, and the needle retract slide 1110. Accordingly, this movement can retract the microneedles relative to the pins (e.g., pin 2052), thus exposing the grafts and positioning the components for a scatter sequence.

In some configurations, the vertical component assembly 1046 can have a "scatter" configuration corresponding to an advanced needle position. During this scatter configuration, for example, the actuator plunger bar 1106 can advance, and the needle retract slide 1110 can advance (similarly, the microneedles can advance). According to some configurations, the actuator plunger bar 1106 can advance, first hitting the top plate 1112, and then hitting the needle plates 2020 (e.g., within microneedle array 2006, see FIG. 5C). This can push the top plate 1112 ahead of needle plates, thus preventing damage to the needle plates 2020. The advancing of the microneedles, followed by the rapid retraction of those microneedles (by the unlocked top plate 1112) can disperse the grafts into the recipient site.

### POWER ON SELF-TEST

In some configurations, the handheld device 1000 can perform a self-test upon start-up (e.g., when the handheld device 1000 is first powered on). In some configurations, the self-test can occur when the handheld device 1000 is plugged in to receive power, and the stand-by input 1018 is pressed and released. The stand-by input 1018 can flash green throughout the duration of the self-test, according to some configurations. Next, the horizontal carriage assembly 1082 can move a very small amount forward, such that the horizontal flag sensor 1064 is cleared. Subsequently, the horizontal carriage assembly 1082 can return to the home position.

During the self-test, the vertical carriage assembly 1108 can move a very small amount upwards, such that the vertical flag 1118 clears the sensor. Subsequently, the vertical carriage assembly 1108 can return to the home position. In some configurations, the vertical carriage assembly 1108 can move up to the unlock position, where it can move the needle retract slide latches 1116, before returning to the home position. This can, for example, release the needle retract slide 1110, in the event that it is locked (e.g., cartridge assembly 2000 is locked in).

In some configurations, the horizontal carriage assembly 1082 can move to a predetermined position (e.g., approximately two-thirds of the way through its full range), which can verify that a cartridge (e.g., cartridge assembly 2000) is not present. Subsequently, the horizontal carriage assembly 1082 can return to the home position.

During the self-test, the flippers 1074 can extend out and then retract back in. Further, in some configurations, some or all lights on handheld device 1000 can flash (e.g., indicator light 1016, 1020, scatter input 1022, etc.). Upon completion of the self-test, the stand-by input 1018 can light up solid green, for example, which can indicate that the self-test was successful.

### CARTRIDGE LOADING AND INITIALIZATION

In some configurations, the skin grafting system 100 can have a predefined cartridge loading and initialization process. The user can open the loading door 1004, then slide the cartridge assembly 2000 (i.e., including the cartridge cover 2004) into the engagement slot 1002. The cartridge latch 1114 can lock onto the cartridge assembly 2000. The user can then remove the cartridge cover 2004 and close the loading door 1004, which can activate the internal loading door switch.

The initialization process can further include moving the horizontal carriage assembly 1082 from the home position, such that it can detect the cartridge presence by stalling on the first needle plate. Subsequently, the horizontal carriage assembly 1082 can return to the home position. Additionally, the vertical carriage assembly 1108 can move a small amount, such that the vertical flag 1118 clears the sensor, and then the vertical carriage assembly 1108 can return to the home position.

In some configurations, the flippers 1074 can extend out above the top plate 1112. The vertical carriage assembly 1108 can move to the lock position. While moving to the lock position, the flippers 1074 can hold the top plate 1112 in place while the needle retract slide latches 1116 move out, and eventually lock over the top plate 1112. Accordingly, the needle retract springs 1120 can be held in a compressed state. While this is happening, for example, the latches on the lockdown latch assemblies (e.g., any configuration of the lockdown latch assembly 1126 described herein) can spring out under the arms 2022 of the needle plates 2020 (e.g., within the microneedle array 2006, see FIG. 5C), in preparation for locking the needle plates 2020 down during the harvest sequence. In some configurations, the vertical carriage assembly 1108 can then move a small amount down, thus moving into the lock relax position (described above). Additionally, the flippers 1074 can retract back in.

The initialization process can further include returning the vertical carriage assembly 1108 to the harvest position. The horizontal carriage assembly 1082 can engage with the first needle plate (within microneedle array 2006) by stalling against the first needle plate and subsequently backing off by a small predetermined distance. The handheld device 1000 can then calculate the position of each needle plate 2020 of the plurality of needle plates. Upon completion of the initialization process, the indicator light 1020 can illuminate white to indicate that the handheld device 1000 is ready for the harvest sequence.

### METHODS OF HARVEST AND EXTRACTION

In some configurations, a user can harvest and extract tissue columns using a harvesting process. The user can position the handheld device 1000 at the donor site, with the tissue stabilizer 2014 pressed against the skin. The user can use one or two hands to apply force against the skin via the handheld device 1000. The tissue stabilizer interface components can move upward, compressing the position sensing springs 1056 until the position sensing flag 1062 occludes the flag sensor. In some configurations, the indicator lights 1016, 1020 can illuminate green, thus indicating that the trigger 1014 is active.

Once the trigger 1014 is active, the user can pull the trigger 1014 (while maintaining sufficient force on the skin) and the handheld device 1000 can begin the harvest sequence. In some configurations, the indicator lights 1016, 1020 can blink green throughout the duration of the harvest and the extraction. The position sensing flag 1062 can be monitored throughout the harvest (between actuator activations) to ensure that sufficient force is maintained. The actuator 1052 can rapidly advance the actuator plunger bar 1106, which can advance the two hammers 1098a, 1098b, and insert the first needle plate into the tissue. The needle plate travels past the needle plate lockdown latches as it is inserted. Subsequently, the actuator 1052 and hammers 1098a, 1098b can retract, and the needle segment can remain locked down in the tissue.

In some configurations, the horizontal carriage assembly 1082 can advance to the calculated position of the next needle segment. Alternatively, the position of the next needle segment can be recalculated or otherwise re-verified throughout the harvest process. The actuator 1052 can rapidly advance the actuator plunger bar 1106, which can advance the two hammers 1098a, 1098b, and insert the next needle plate into the tissue. The needle plate can travel past the latches on the lockdown latch assembly (e.g., any configuration of the lockdown latch assembly 1126 described herein) as it is inserted. The latches can then spring back out, and the actuator 1052 and hammers 1098a, 1098b can retract. This insertion process can repeat until all needle segments have been inserted into the tissue.

After completing the insertion of all segments, the horizontal carriage assembly 1082 can return to the home position, according to some configurations. The vertical carriage assembly 1108 can move up to the extraction position, extracting the microneedles from the donor tissue, and positioning the microneedles safely up inside the tissue stabilizer 2014. The indicator lights 1016, 1020 can stop blinking green and turn off. Additionally, the scatter input 1022 can be illuminated white, indicating that the handheld device 1000 is ready to proceed with the scattering process. Upon completion of the harvesting process, the user can remove the force on the tissue, and lift the handheld device 1000 away.

### METHODS OF SCATTER

In some configurations, a user can scatter the tissue columns after the harvesting process. Once the user has removed the handheld device 1000 from the donor site (with the tissue columns harvested), the microneedles can be safely up inside of the cartridge housing 2002 (e.g., within the tissue stabilizer 2014). With the recipient site ready for the tissue columns, the user can activate the scatter mode by pressing the scatter input 1022. In some configurations, the scatter input 1022 can change from being illuminated white to green.

In some configurations, the user can position the cartridge assembly 2000 directly above the recipient site. The user can then pull the trigger 1014 and the vertical carriage assembly 1108 can move out of the extract position, which can release the needle retract slide 1110 and retract the microneedles behind the pins (e.g., pins 2052). The handheld device 1000 can rapidly advance the actuator plunger bar 1106 which accordingly pushes both the needle retract slide 1110 and the needle plates. The needle retract slide 1110 can remain pushed ahead of the needle plates to prevent damage to the needle plates. Subsequently, the actuator plunger bar 1106 can retract, which can cause the needle retract slide 1110 to retract (pulling the needle plates back with the needle retract slide 1110). The process of rapidly advancing the actuator plunger bar 1106 can be repeated a plurality of times, which can ensure that as many grafts as possible have been deposited into the recipient site. In some configurations, six activations of the actuator 1052 can occur. In other configurations, three activations of the actuator 1052 can occur. After the scatter process has completed, the vertical carriage assembly 1108 can return to the home position, with the needle retract slide 1110 unlocked.

### CARTRIDGE REMOVAL

In some configurations, once the user has completed the harvest and scatter processes, the user can open the loading door 1004, depress the cartridge latch 1114, and slide the cartridge assembly 2000 out. In some configurations, if the user wants to complete another harvest with the same cartridge assembly 2000, the user can open and close the loading door 1004 (i.e., *without* removing the cartridge assembly 2000). Opening and closing of the loading door 1004 can begin another initialization process via the handheld device 1000. Alternatively, the user can begin another initialization process via an input (not shown) on the user interface 1008.

While the present disclosure may be susceptible to various modifications and alternative forms, specific configurations have been shown by way of example in the drawings and have been described in detail herein. However, it should be understood that the present disclosure is not intended to be limited to the particular forms disclosed.

This written description uses examples to disclose the present disclosure, including the best mode, and also to enable any person skilled in the art to practice the present disclosure, including making and using any devices or systems and performing any incorporated methods. The patentable scope of the present disclosure is defined by the claims and may include other examples that occur to those skilled in the art. Such other examples are intended to be within the scope of the claims if they have structural elements that do not differ from the literal language of the claims.

Finally, it is expressly contemplated that any of the processes or steps described herein may be combined, eliminated, or reordered. Accordingly, this description is meant to be taken only by way of example, and not to otherwise limit the scope of this present disclosure.

## Claims

1. A skin grafting system (100) comprising:
a plurality of hollow microneedles (2050), wherein the microneedles (2050) are actuatable between a retracted position and an extended position;
a rigid member (2022) coupled to the plurality of hollow microneedles; and
a latch assembly (1126) comprising:
a pair of latches (3006, 3008) coupled to the latch assembly,
wherein at least one latch (3006, 3008) of the pair of latches (3006, 3008) is configured to inhibit movement of the rigid member (2022) when the plurality of microneedles (2050) are in the extended position,
**characterized in that** the latch assembly (1126) comprises:
a body (3002) centrally disposed between the pair of latches (3006, 3008), and wherein the latches corresponding to the pair of latches (3006, 3008) are horizontally opposed about the body (3002); and
a spring (3010) disposed between the pair of latches (3006, 3008) and wherein preferably the spring (3010) is retained within the body (3002).

2. The skin grafting system (100) of claim 1, wherein
**(a)** at least one latch (3006, 3008) is movable during actuation of the plurality of hollow microneedles (2050) and is pivotally coupled to the latch assembly (1126); and/or
(b) the pair of latches is configured to engage the rigid member (2022).

3. The skin grafting system (100) of claim 1, wherein the at least one latch (3006, 3008) is movable during actuation of the plurality of hollow microneedles (2050) and is slidably coupled to the latch assembly (1126), wherein preferably the latches are configured to slide horizontally between a latched position and an unlatched position, the latched position corresponding to the plurality of hollow microneedles (2050) being in the extended position.

4. The skin grafting system (100) of claim 1, wherein the spring (3010) is configured to bias the latch (3006, 3008) to the latched position.

5. The skin grafting system (100) of claim 1, wherein the spring (5010) is a double torsion spring arranged between the pair of latches (5006, 5008) and configured to bias the latches to a latched position.

6. The skin grafting system (100) of claim 1, further comprising a guide plate (6068) configured to engage with a guide profile (6070) formed into the pair of latches (6006, 6008) such that the latches slide horizontally between a latched position and an unlatched position.

7. The skin grafting system (100) of claim 6, further comprising a shaft (6072) coupled to the guide plate (6068), wherein the shaft (6072) is slidably coupled to the body (6002) via a shaft aperture (6075) within the body (6002);
wherein preferably a coil spring (6074) of the skin grafting system (100) is disposed within the shaft (6072) and configured to bias the guide plate (6068) upwards such that the latches remain in the latched position.

8. The skin grafting system (100) of any one of the preceding claims, further comprising:
a carrier (2020) actuatable between a retracted position and an extended position;
wherein the plurality of microneedles (2050) are coupled to the carrier (2020) and configured to extract tissue cores from a donor site as the carrier (2020) moves from a retracted position to an extended position and back to a retracted position;
wherein the latch is configured to move between a plurality of positions, including a latched position restricting movement of the carrier (2020) from the extended position to the retracted position to thereby lock the plurality of hollow microneedles (2050) in a position configured to engage the donor site; and
wherein preferably a rigid member (2022) of the skin grafting system (100) is coupled to the carrier (2020) and configured to be engaged by the latch to restrict movement of the carrier (2020) to the retracted position upon the plurality of hollow microneedles (2050) arriving in the extended position.

9. The skin grafting system (100) of claim 8, further comprising:
a handheld device (1000) including the latch;
a cartridge assembly (2000) including the plurality of microneedles (2050) coupled to the carrier (2020),
wherein the cartridge assembly (2000) is configured to be removably attached to the handheld device (1000), and
wherein the handheld device (1000) is configured to engage with the cartridge assembly (2000) during a skin grafting process.

10. The skin grafting system (100) of claim 9, wherein the handheld device (1000) is configured to automatically control movement of the latch among the plurality of positions during the skin grafting process.

11. The skin grafting system (100) of claim 1, wherein the body (3002) comprises pin apertures (3028), each latch including a proximal end configured to couple to the body (3002) via at least one pivot pin (3020) inserted through the pin apertures (3028).

12. The skin grafting system (100) of claim 1, wherein each latch includes a protrusion (3036) configured to inhibit movement of the rigid member (2022) when in the latched position.

13. The skin grafting system (100) of claim 1, wherein a latched position of the latch is associated with an extended position of the plurality of microneedles (2050), the plurality of microneedles (2050) positioned within donor tissue.

14. The skin grafting system (100) of claim 1, wherein the latch assembly (1126) includes a plurality of first latches (3006) and a corresponding plurality of second latches (3008), wherein the first and second latches (3006, 3008) are arranged in complementary pairs on opposing sides of the latch assembly (1126);
wherein the plurality of microneedles (2050) forms a microneedle array (2006), the microneedle array (2006) being formed by assembling a plurality of rows of microneedles (250), and wherein each row of microneedles can be separately and sequentially actuated; and
wherein the latch assembly (1126) is configured to lock each row of microneedles (2050) in the microneedle array (2006) in the extended position after being actuated from the retraced position to the extended position.

15. The skin grafting system (100) of claim 1, wherein each microneedle (2050) of the plurality of microneedles (2050) is configured to facilitate harvesting of tissue cores from a donor site.

## Patentansprüche

1. Ein Hauttransplantationssystem (100), umfassend:
eine Vielzahl von hohlen Mikronadeln (2050), wobei die Mikronadeln (2050) zwischen einer zurückgezogenen Position und einer ausgefahrenen Position betätigbar sind;
ein starres Element (2022), das mit der Vielzahl von hohlen Mikronadeln gekoppelt ist; und
eine Verriegelungsbaugruppe (1126), umfassend:
ein Paar von Riegeln (3006, 3008), die mit der Verriegelungsbaugruppe gekoppelt sind,
wobei mindestens ein Riegel (3006, 3008) des Paares von Riegeln (3006, 3008) dazu konfiguriert ist, eine Bewegung des starren Elements (2022) zu hemmen, wenn sich die Vielzahl von Mikronadeln (2050) in der ausgefahrenen Position befinden,
**dadurch gekennzeichnet, dass** die Verriegelungsbaugruppe (1126) umfasst:
einen Körper (3002), der mittig zwischen dem Paar von Riegeln (3006, 3008) angeordnet ist, und wobei die dem Paar von Riegeln (3006, 3008) entsprechenden Riegel bezüglich des Körpers (3002) horizontal einander gegenüberliegend angeordnet sind; und
eine Feder (3010), die zwischen dem Paar von Riegeln (3006, 3008) angeordnet ist, und wobei die Feder (3010) vorzugsweise innerhalb des Körpers (3002) gehalten ist.

2. Das Hauttransplantationssystem (100) nach Anspruch 1, wobei
(a) mindestens ein Riegel (3006, 3008) während einer Betätigung der Vielzahl von hohlen Mikronadeln (2050) beweglich ist und schwenkbar mit der Verriegelungsbaugruppe (1126) gekoppelt ist; und/oder
(b) das Paar von Riegeln dazu konfiguriert ist, mit dem starren Element (2022) in Eingriff zu treten.

3. Das Hauttransplantationssystem (100) nach Anspruch 1, wobei der mindestens eine Riegel (3006, 3008) während einer Betätigung der Vielzahl von hohlen Mikronadeln (2050) beweglich ist und verschiebbar mit der Verriegelungsbaugruppe (1126) gekoppelt ist, wobei die Riegel vorzugsweise dazu konfiguriert sind, horizontal zwischen einer verriegelten Position und einer entriegelten Position zu gleiten, wobei die verriegelte Position der Vielzahl von hohlen Mikronadeln (2050) in der ausgefahrenen Position entspricht.

4. Das Hauttransplantationssystem (100) nach Anspruch 1, wobei die Feder (3010) dazu konfiguriert ist, den Riegel (3006, 3008) in die verriegelte Position vorzuspannen.

5. Das Hauttransplantationssystem (100) nach Anspruch 1, wobei die Feder (5010) eine Doppeltorsionsfeder ist, die zwischen dem Paar von Riegeln (5006, 5008) angeordnet und dazu konfiguriert ist, die Riegel in eine verriegelte Position vorzuspannen.

6. Das Hauttransplantationssystem (100) nach Anspruch 1, ferner umfassend eine Führungsplatte (6068), die dazu konfiguriert ist, mit einem in dem Paar von Riegeln (6006, 6008) ausgebildeten Führungsprofil (6070) in Eingriff zu treten, sodass die Riegel horizontal zwischen einer verriegelten Position und einer entriegelten Position gleiten.

7. Das Hauttransplantationssystem (100) nach Anspruch 6, ferner umfassend einen Schaft (6072), der mit der Führungsplatte (6068) gekoppelt ist, wobei der Schaft (6072) über eine Schaftöffnung (6075) innerhalb des Körpers (6002) verschiebbar mit dem Körper (6002) gekoppelt ist;
wobei vorzugsweise eine Schraubenfeder (6074) des Hauttransplantationssystems (100) innerhalb des Schafts (6072) angeordnet und dazu konfiguriert ist, die Führungsplatte (6068) nach oben vorzuspannen, sodass die Riegel in der verriegelten Position bleiben.

8. Das Hauttransplantationssystem (100) nach einem der vorhergehenden Ansprüche, ferner umfassend:
einen Träger (2020), der zwischen einer zurückgezogenen Position und einer ausgefahrenen Position betätigbar ist;
wobei die Vielzahl von Mikronadeln (2050) mit dem Träger (2020) gekoppelt und dazu konfiguriert sind, Gewebekerne aus einer Spenderstelle zu entnehmen, während sich der Träger (2020) von einer zurückgezogenen Position in eine ausgefahrene Position und zurück in eine zurückgezogene Position bewegt;
wobei der Riegel dazu konfiguriert ist, sich zwischen einer Vielzahl von Positionen zu bewegen, einschließlich einer verriegelten Position, die eine Bewegung des Trägers (2020) von der ausgefahrenen Position in die zurückgezogene Position beschränkt, um dadurch die Vielzahl von hohlen Mikronadeln (2050) in einer Position zu verriegeln, die dazu konfiguriert ist, mit der Spenderstelle in Eingriff zu treten; und
wobei vorzugsweise ein starres Element (2022) des Hauttransplantationssystems (100) mit dem Träger (2020) gekoppelt und dazu konfiguriert ist, von dem Riegel in Eingriff genommen zu werden, um eine Bewegung des Trägers (2020) in die zurückgezogene Position zu beschränken, sobald die Vielzahl von hohlen Mikronadeln (2050) die ausgefahrene Position erreicht hat.

9. Das Hauttransplantationssystem (100) nach Anspruch 8, ferner umfassend:
ein Handgerät (1000), das den Riegel aufweist;
eine Kartuschenbaugruppe (2000), die die mit dem Träger (2020) gekoppelte Vielzahl von Mikronadeln (2050) aufweist,
wobei die Kartuschenbaugruppe (2000) dazu konfiguriert ist, lösbar an dem Handgerät (1000) befestigt zu werden, und
wobei das Handgerät (1000) dazu konfiguriert ist, während eines Hauttransplantationsvorgangs mit der Kartuschenbaugruppe (2000) in Eingriff zu treten.

10. Das Hauttransplantationssystem (100) nach Anspruch 9, wobei das Handgerät (1000) dazu konfiguriert ist, eine Bewegung des Riegels zwischen der Vielzahl von Positionen während des Hauttransplantationsvorgangs automatisch zu steuern.

11. Das Hauttransplantationssystem (100) nach Anspruch 1, wobei der Körper (3002) Stiftöffnungen (3028) umfasst, wobei jeder Riegel ein proximales Ende aufweist, das dazu konfiguriert ist, über mindestens einen durch die Stiftöffnungen (3028) eingeführten Schwenkstift (3020) mit dem Körper (3002) gekoppelt zu werden.

12. Das Hauttransplantationssystem (100) nach Anspruch 1, wobei jeder Riegel einen Vorsprung (3036) aufweist, der dazu konfiguriert ist, in der verriegelten Position eine Bewegung des starren Elements (2022) zu hemmen.

13. Das Hauttransplantationssystem (100) nach Anspruch 1, wobei eine verriegelte Position des Riegels einer ausgefahrenen Position der Vielzahl von Mikronadeln (2050) zugeordnet ist, wobei die Vielzahl von Mikronadeln (2050) innerhalb von Spendergewebe positioniert sind.

14. Das Hauttransplantationssystem (100) nach Anspruch 1, wobei die Verriegelungsbaugruppe (1126) eine Vielzahl von ersten Riegeln (3006) und eine entsprechende Vielzahl von zweiten Riegeln (3008) aufweist, wobei die ersten und zweiten Riegel (3006, 3008) in komplementären Paaren auf gegenüberliegenden Seiten der Verriegelungsbaugruppe (1126) angeordnet sind;
wobei die Vielzahl von Mikronadeln (2050) ein Mikronadelarray (2006) bildet, wobei das Mikronadelarray (2006) durch Zusammenfügen einer Vielzahl von Reihen von Mikronadeln (250) gebildet ist, und wobei jede Reihe von Mikronadeln separat und nacheinander betätigt werden kann; und
wobei die Verriegelungsbaugruppe (1126) dazu konfiguriert ist, jede Reihe von Mikronadeln (2050) in dem Mikronadelarray (2006) in der ausgefahrenen Position zu verriegeln, nachdem sie von der zurückgezogenen Position in die ausgefahrene Position betätigt worden ist.

15. Das Hauttransplantationssystem (100) nach Anspruch 1, wobei jede Mikronadel (2050) der Vielzahl von Mikronadeln (2050) dazu konfiguriert ist, eine Gewinnung von Gewebekernen aus einer Spenderstelle zu erleichtern.

## Revendications

1. Système de greffe de peau (100) comprenant :
une pluralité de micro-aiguilles creuses (2050), dans lequel les micro-aiguilles (2050) peuvent être actionnées entre une position rétractée et une position déployée ;
un élément rigide (2022) accouplé à la pluralité de micro-aiguilles creuses ; et
un ensemble de verrouillage (1126) comprenant :
une paire de verrous (3006, 3008) accouplés à l'ensemble de verrouillage, dans lequel au moins un verrou (3006, 3008) de la paire de verrous (3006, 3008) est configuré pour empêcher un déplacement de l'élément rigide (2022) lorsque la pluralité de micro-aiguilles (2050) se trouvent dans la position déployée,
**caractérisé en ce que** l'ensemble de verrouillage (1126) comprend :
un corps (3002) disposé centralement entre la paire de verrous (3006, 3008), et dans lequel les verrous correspondant à la paire de verrous (3006, 3008) sont horizontalement opposés de part et d'autre du corps (3002) ; et
un ressort (3010) disposé entre la paire de verrous (3006, 3008), et dans lequel, de préférence, le ressort (3010) est retenu à l'intérieur du corps (3002).

2. Système de greffe de peau (100) selon la revendication 1, dans lequel :
(a) au moins un verrou (3006, 3008) est mobile pendant l'actionnement de la pluralité de micro-aiguilles creuses (2050) et est accouplé de manière pivotante à l'ensemble de verrouillage (1126) ; et/ou
(b) la paire de verrous est configurée pour venir en prise avec l'élément rigide (2022).

3. Système de greffe de peau (100) selon la revendication 1, dans lequel l'au moins un verrou (3006, 3008) est mobile pendant l'actionnement de la pluralité de micro-aiguilles creuses (2050) et est accouplé de manière coulissante à l'ensemble de verrouillage (1126), dans lequel, de préférence, les verrous sont configurés pour coulisser horizontalement entre une position verrouillée et une position déverrouillée, la position verrouillée correspondant à la pluralité de micro-aiguilles creuses (2050) se trouvant dans la position déployée.

4. Système de greffe de peau (100) selon la revendication 1, dans lequel le ressort (3010) est configuré pour solliciter le verrou (3006, 3008) vers la position verrouillée.

5. Système de greffe de peau (100) selon la revendication 1, dans lequel le ressort (5010) est un ressort à double torsion agencé entre la paire de verrous (5006, 5008) et configuré pour solliciter les verrous vers une position verrouillée.

6. Système de greffe de peau (100) selon la revendication 1, comprenant en outre une plaque de guidage (6068) configurée pour venir en prise avec un profil de guidage (6070) formé dans la paire de verrous (6006, 6008), de telle sorte que les verrous coulissent horizontalement entre une position verrouillée et une position déverrouillée.

7. Système de greffe de peau (100) selon la revendication 6, comprenant en outre une tige (6072) accouplée à la plaque de guidage (6068), dans lequel la tige (6072) est accouplée de manière coulissante au corps (6002) par l'intermédiaire d'une ouverture de tige (6075) dans le corps (6002) ;
dans lequel, de préférence, un ressort hélicoïdal (6074) du système de greffe de peau (100) est disposé à l'intérieur de la tige (6072) et configuré pour solliciter la plaque de guidage (6068) vers le haut, de telle sorte que les verrous restent dans la position verrouillée.

8. Système de greffe de peau (100) selon l'une quelconque des revendications précédentes, comprenant en outre :
un support (2020) pouvant être actionné entre une position rétractée et une position déployée ;
dans lequel la pluralité de micro-aiguilles (2050) sont accouplées au support (2020) et configurée pour extraire des carottes de tissu d'un site donneur lorsque le support (2020) passe d'une position rétractée à une position déployée, puis revient à une position rétractée ;
dans lequel le verrou est configuré pour se déplacer entre une pluralité de positions, comportant une position verrouillée limitant le déplacement du support (2020) de la position déployée à la position rétractée, afin de verrouiller ainsi la pluralité de micro-aiguilles creuses (2050) dans une position configurée pour venir en prise avec le site donneur ; et
dans lequel, de préférence, un élément rigide (2022) du système de greffe de peau (100) est accouplé au support (2020) et configuré pour être mis en prise par le verrou afin de limiter le déplacement du support (2020) vers la position rétractée lorsque la pluralité de micro-aiguilles creuses (2050) atteignent la position déployée.

9. Système de greffe de peau (100) selon la revendication 8, comprenant en outre :
un dispositif portatif (1000) comportant le verrou ;
un ensemble cartouche (2000) comportant la pluralité de micro-aiguilles (2050) accouplées au support (2020),
dans lequel l'ensemble cartouche (2000) est configuré pour être fixé de manière amovible au dispositif portatif (1000), et
dans lequel le dispositif portatif (1000) est configuré pour venir en prise avec l'ensemble cartouche (2000) pendant un processus de greffe de peau.

10. Système de greffe de peau (100) selon la revendication 9, dans lequel le dispositif portatif (1000) est configuré pour commander automatiquement le déplacement du verrou parmi la pluralité de positions pendant le processus de greffe de peau.

11. Système de greffe de peau (100) selon la revendication 1, dans lequel le corps (3002) comprend des ouvertures de broche (3028), chaque verrou comportant une extrémité proximale configurée pour être accouplée au corps (3002) par l'intermédiaire d'au moins une broche de pivotement (3020) insérée à travers les ouvertures de broche (3028).

12. Système de greffe de peau (100) selon la revendication 1, dans lequel chaque verrou comporte une saillie (3036) configurée pour empêcher le déplacement de l'élément rigide (2022) lorsqu'il se trouve dans la position verrouillée.

13. Système de greffe de peau (100) selon la revendication 1, dans lequel une position verrouillée du verrou est associée à une position déployée de la pluralité de micro-aiguilles (2050), la pluralité de micro-aiguilles (2050) étant positionnées à l'intérieur d'un tissu donneur.

14. Système de greffe de peau (100) selon la revendication 1, dans lequel l'ensemble de verrouillage (1126) comporte une pluralité de premiers verrous (3006) et une pluralité correspondante de deuxièmes verrous (3008), dans lequel les premiers et deuxièmes verrous (3006, 3008) sont agencés en paires complémentaires sur des côtés opposés de l'ensemble de verrouillage (1126) ;
dans lequel la pluralité de micro-aiguilles (2050) forment un réseau de micro-aiguilles (2006), le réseau de micro-aiguilles (2006) étant formé par l'assemblage d'une pluralité de rangées de micro-aiguilles (250), et dans lequel chaque rangée de micro-aiguilles peut être actionnée séparément et séquentiellement ; et
dans lequel l'ensemble de verrouillage (1126) est configuré pour verrouiller chaque rangée de micro-aiguilles (2050) du réseau de micro-aiguilles (2006) dans la position déployée après son actionnement de la position rétractée à la position déployée.

15. Système de greffe de peau (100) selon la revendication 1, dans lequel chaque micro-aiguille (2050) de la pluralité de micro-aiguilles (2050) est configurée pour faciliter le prélèvement de carottes de tissu sur un site donneur.
